# EUROPÄISCHE PATENTSCHRIFT

(11) **EP 3 792 340 B1**
(45) Veröffentlichungstag und Bekanntmachung des Hinweises auf die Patenterteilung: **15.09.2021**
(21) Anmeldenummer: 20192381.0
(22) Anmeldetag: 24.08.2020
(51) Int. Cl.: C12M 1/107, C12M 1/00, C12M 3/00, C12M 1/06, C12M 1/34

(54) **GASDICHTER BEHÄLTER**
GAS-TIGHT CONTAINER
RÉSERVOIR ÉTANCHE AU GAZ

(30) Priorität: 04.09.2019 DE 102019123653; 21.07.2020 DE 102020119254
(43) Veröffentlichungstag der Anmeldung: 17.03.2021
(73) Patentinhaber: XL Beteiligungen GmbH & Co. KG, 73497 Tannhausen (DE)
(72) Erfinder: Lipp, Manuel, 73479 Ellwangen (DE); Göggerle, Michael, 73497 Tannhausen (DE)
(74) Vertreter: Lorenz, Markus

(56) Entgegenhaltungen:
- EP-A1- 0 516 580
- EP-A1- 1 918 621
- DE-A1- 19 954 904
- DE-A1-102006 026 118
- DE-A1-102009 059 262
- DE-A1-102012 110 812

## Beschreibung

Die Erfindung betrifft einen gasdichten Behälter, insbesondere ein Silo zur Lagerung von organischen Substanzen, aufweisend einen Aufnahmeraum, einen Boden, eine umlaufende Wandung und eine Dacheinrichtung, wobei die Dacheinrichtung eine gasdichte Abdeckung aufweist, welche mit der Wandung gasdicht verbunden ist.

Bei dem gasdichten Behälter handelt es sich vorzugsweise um einen gasdichten Großraumbehälter.

Die Erfindung betrifft auch eine Vorrichtung zur Gewinnung eines gasförmigen Energieträgers aus organischen Substanzen, insbesondere einen Biogasreaktor, aufweisend einen gasdichten Behälter.

Ein gattungsgemäßer gasdichter Behälter ist beispielsweise aus der DE 42 32 318 A1 bekannt.

Aus der DE 199 54 904 A1 ist ferner eine Vorrichtung zur Gewinnung eines gasförmigen Energieträgers aus organischen Substanzen bekannt.

Generell ist es aus dem Stand der Technik für verschiedene Anwendungen bekannt, gasdichte Behälter zur Aufnahme von flüssigen, gasförmigen oder festen Stoffen einzusetzen.

Insbesondere in der Industrie und auch bei Kommunen besteht ein Bedarf an Großraumbehältern bzw. an großvolumigen Behältern, die beispielsweise ein Volumen von 100 bis 50.000 m³ aufweisen können, um Flüssigkeiten, beispielsweise auch Abwässer und Schlämme oder Zwischen- und Endprodukte der chemischen, pharmazeutischen und nahrungsmittelverarbeitenden Industrie aufzunehmen. Ferner besteht auch ein Bedarf an Gasspeichern, insbesondere zur Lagerung von Biogas, Klärgas oder Deponiegas.

Des Weiteren besteht ein Bedarf an Behältern, insbesondere an Silos, zur Lagerung von organischen Substanzen bzw. zur Lagerung von pflanzlichen Produkten.

Bekannt sind auch sogenannte Biogasreaktoren, die auch als Fermenter bezeichnet werden, bei denen es sich um Behälter handelt, in denen Mikroorganismen, Zellen oder kleine Pflanzen kultiviert werden. Bekannt sind dabei insbesondere Biogasreaktoren von Biogasanlagen, bei denen die Biomasse in einem anaeroben Prozess zu Biogas und Gärresten abgebaut wird. Derartige Behälter sind luft- bzw. gasdicht abgeschlossen und verfügen über ein Rührwerk und verschiedene Mess-, Steuer- und Regelungseinrichtungen (MSR) zur Prozesskontrolle.

Aus der DE 197 56 485 A1 ist eine Vorrichtung bekannt, bei der beispielsweise Fäkalien in einem Gärraum im Durchschnitt mehrere Tage bei einer Temperatur von beispielsweise etwa 35° eingelagert und das entstehende Faulgas in einem über dem Gärraum angeordneten Gasraum aufgefangen wird. Der Gasraum ist nach oben von einer gasdichten Hülle begrenzt, die innerhalb des Gasraums an einer den Gärraum bildenden Wandung festgelegt ist.

Eine besondere Anforderung bei der Herstellung von großvolumigen Behältern, insbesondere wenn diese als Biogasreaktor von Biogasanlagen bzw. als Fermenter eingesetzt werden, besteht darin, dass die Konstruktion gasdicht ausgebildet sein soll. Diese Anforderung besteht dabei entsprechend auch für die Dacheinrichtung bzw. für die Dachkonstruktion eines derartigen Behälters.

Die aus dem Stand der Technik bekannten üblichen Dacheinrichtungen, beispielsweise ein Kegeldach, so wie dies z. B. in der DE 199 54 904 A1 gezeigt ist, lassen sich nicht oder technisch nur sehr aufwändig gasdicht herstellen. Dies liegt unter anderem an den notwendigen Verschraubungen des Kegeldachs selbst bzw. dessen Anbindung an die Wandung des Behälters.

Diesbezüglich wird beispielsweise in der DE 199 54 904 A1 vorgeschlagen, eine zusätzliche Innenhülle zu verwenden.

Aus dem Stand der Technik ist es ferner bekannt, dass die Dacheinrichtung eine Abdeckung aufweist, die aus flachen Metallbahnen gebildet ist. Eine derartige Konstruktion wird auch als Membranabdeckung bezeichnet. Die Metallbahnen, aus denen die Abdeckung gebildet ist, werden dabei gasdicht miteinander verschweißt, um eine einheitliche, gasdichte Abdeckung auszubilden. Aus Kosten- und Gewichtsgründen ist die derart gebildete Abdeckung zumeist aus Metallbahnen gebildet, die eine vergleichsweise geringe Stärke aufweisen. Um die Abdeckung vor Witterungseinflüssen und auch vor Beschädigungen zu schützen, ist es aus dem Stand der Technik zusätzlich bekannt, dass oberhalb der Abdeckung ein Kegeldach ausgebildet ist, das selbst nicht gasdicht ist.

Eine derartige Konstruktion hat sich in der Praxis bewährt.

Es hat sich allerdings gezeigt, dass die aus den flachen, dünnen Metallbahnen gebildete Abdeckung, d. h. das Membrandach, zu einem "Flattern" neigt, wenn der Aufnahmeraum des Behälters unter Druck gesetzt wird.

Bei der Produktion von Gas bzw. Biogas kann, außergewöhnliche Umstände vorausgesetzt, eine Explosionsgefahr bestehen. Eine derartige Explosionsgefahr gilt es soweit möglich zu reduzieren bzw. Maßnahmen zu ergreifen, dass im Falle einer Explosion die auftretenden Schäden möglichst gering sind.

Der vorliegenden Erfindung liegt daher die Aufgabe zugrunde, die aus dem Stand der Technik bekannten gasdichten Behälter zu verbessern, insbesondere die Sicherheit der Behälter zu erhöhen.

Der vorliegenden Erfindung liegt auch die Aufgabe zugrunde, eine Vorrichtung zur Gewinnung eines gasförmigen Energieträgers aus organischen Substanzen, insbesondere einen Biogasreaktor, zur Verfügung zu stellen, der gegenüber dem Stand der Technik verbessert ist.

Die Aufgabe wird hinsichtlich des gasdichten Behälters durch die Merkmale des Anspruchs 1 gelöst.

Die Aufgabe wird ferner hinsichtlich der Vorrichtung zur Gewinnung eines gasförmigen Energieträgers aus organischen Substanzen durch die Merkmale des Anspruchs 15 gelöst.

Die Erfindung betrifft einen gasdichten Behälter, insbesondere ein Silo, zur Lagerung von organischen Substanzen. Der erfindungsgemäße Behälter weist einen Aufnahmeraum, einen Boden, eine umlaufende Wandung und eine Dacheinrichtung auf. Die Dacheinrichtung weist eine gasdichte Abdeckung auf.

Der erfindungsgemäße Behälter ist vorzugsweise ein Großraumbehälter.

Erfindungsgemäß ist vorgesehen, dass zwischen der Abdeckung und der Wandung des Behälters ein Berstelement eingesetzt ist, welches beim Erreichen eines definierten Berstdrucks die gasdichte Verbindung zwischen der Abdeckung und der Wandung löst.

Der Erfinder hat erkannt, dass sich die Sicherheit derartiger gasdichter Behälter signifikant erhöhen lässt, wenn ein Berstelement eingesetzt ist, welches beim Erreichen eines definierten Berstdrucks die gasdichte Verbindung zwischen der Abdeckung und der Wandung löst.

Die Verbindung zwischen der Abdeckung und der Wandung kann dabei teilweise, vorzugsweise jedoch vollständig, insbesondere ringförmig umlaufend, gelöst werden.

Die vorgenannte Ausgestaltung, die bewirkt, dass sich die gasdichte Verbindung zwischen der Wandung und der Abdeckung löst, wenn der Druck im Aufnahmeraum einen definierten Berstdruck erreicht bzw. übersteigt, erhöht die Sicherheit von gasdichten Behältern, insbesondere wenn diese als Biogasreaktoren bzw. Fermenter eingesetzt werden, deutlich. Bei der Produktion von Gas bzw. Biogas kann, wie bereits erwähnt, beim Vorliegen außergewöhnlicher Umstände, die Gefahr bestehen, dass es zu einer Explosion des Behälters kommt. Der Erfinder hat erkannt, dass sich eine Gefährdungssituation durch eine Gasexplosion erheblich reduzieren lässt, wenn sich die gasdichte Verbindung zwischen der Wandung und der Abdeckung löst, wenn sich in dem Aufnahmeraum ein Druck aufbaut, der dem definierten Berstdruck entspricht bzw. über dem Berstdruck liegt, insbesondere wenn sich ein Überdruck von mehr als 100 mbar aufbaut. Eine besonders hohe Sicherheit kann sich ergeben, wenn sich die Verbindung bereits löst, wenn sich ein Überdruck von mehr als 80 mbar, weiter bevorzugt von mehr als 60mbar, ganz besonders bevorzugt von mehr als 50 mbar, insbesondere von mehr als 40mbar aufbaut.

Es kann insbesondere von Vorteil sein, wenn sich die Verbindung bereits bei einem Überdruck von weniger als 50 mbar löst.

Durch das Lösen der Verbindung kann der Druck aus dem Aufnahmeraum in die Atmosphäre, d. h. nach außen, abgegeben werden, so dass sich im Aufnahmeraum im Wesentlichen wieder der Umgebungsdruck oder nur ein geringer Überdruck einstellt. Der Normaldruck, d. h. der mittlere Luftdruck der Atmosphäre beträgt typischerweise 1,01325 bar. D. h., wenn der Bestdruck einem Überdruck von 100 mbar entspricht, ist die Verbindung zwischen der Wandung und der Abdeckung bzw. das Berstelement derart ausgelegt, dass sich die gasdichte Verbindung zwischen der Wandung und der Abdeckung löst, wenn ein absoluter Gasdruck von mehr als 1,1325 bar im Aufnahmeraum, d. h. ein Überdruck von 100 mbar verglichen mit dem Umgebungsdruck herrscht.

Der Berstdruck ist als der Druck zu verstehen, der über dem Umgebungsdruck liegt, der außerhalb des gasdichten Behälters herrscht. Der Druck außerhalb des gasdichten Behälters beträgt im Regelfall 1,01325 bar (Normaldruck), wie vorstehend bereits beschrieben wurde. Der Druck außerhalb des Behälters hängt jedoch auch vom Einbauort des Behälters ab, so dass der Druck außerhalb des Behälters auch höher als der Normaldruck sein kann, entsprechend höher ist dann auch der absolute Gasdruck, bei dem sich die gasdichte Verbindung zwischen der Wandung und der Abdeckung löst.

Es kann, insbesondere bei dem Betrieb eines Biogasreaktors bzw. eines Fermenters, vorgesehen sein, dass der Betriebsdruck in dem Aufnahmeraum derart gewählt wird, dass dieser einem Überdruck von 2 bis 25 mbar entspricht. Es hat sich gezeigt, dass dies für den Betrieb von gasdichten Behältern, insbesondere von Biogasreaktoren und Fermentern, besonders geeignet ist. Zum einen wird durch den geringen Überdruck die Erzeugung von Biogas verbessert, zum anderen unterstützt ein Überdruck im Aufnahmeraum das Entnehmen des Gases aus dem Aufnahmeraum, um das Gas beispielsweise einem externen Gastank zuzuführen. Hierzu weist der Aufnahmeraum in bekannter Weise eine Gasauslassöffnung auf. Dies ist aus dem Stand der Technik bekannt, wozu beispielsweise auf die DE 197 56 485 A1 verwiesen wird. Ein Überdruck im Aufnahmeraum von vorzugsweise 2 bis 25 mbar hat sich auch als geeignet herausgestellt, um zu vermeiden, dass von außen Sauerstoff in den Aufnahmeraum eindringt.

Der für den Betrieb des Fermenters bzw. des Biogasreaktors bzw. allgemein eines gasdichten Behälters vorgesehene geringe Überdruck im Aufnahmeraum von 25 mbar, der auch +/- 20 mbar betragen kann, hat sich als vorteilhaft herausgestellt. Gleichwohl hat es sich als vorteilhaft herausgestellt, wenn bereits bei einem geringen Berstdruck die gasdichte Verbindung zwischen der Wandung und der Abdeckung gelöst wird, um ein Explosionsrisiko bzw. die Schäden, die bei einer Explosion auftreten können, zu reduzieren.

Es kann von Vorteil sein darauf zu achten, dass die Differenz zwischen dem gewünschten Betriebsdruck, d. h. dem für den Betrieb gewünschten Überdruck, und dem Berstdruck, d. h. dem Überdruck, ab dem die gasdichte Verbindung zwischen der Wandung und der Abdeckung gelöst wird, derart gewählt wird, dass der Berstdruck nicht im Betrieb bereits toleranzbedingt erreicht wird.

Die vorgenannten Werte für den Betriebsdruck, insbesondere ein Betriebsdruck von 25 mbar oder weniger, gegebenenfalls auch 30 mbar oder weniger, und einem Berstdruck, der einem Überdruck von 50 mbar oder weniger als 50 mbar, jedoch höher als der Betriebsdruck, entspricht, haben sich als besonders geeignet herausgestellt.

Das Berstelement kann vorzugsweise derart ausgeführt werden, dass die Abdeckung, wenn der Berstdruck überschritten wird, von der Wandung abgehoben wird.

Erfindungsgemäß kann ferner vorgesehen sein, dass das Berstelement derart ausgebildet ist, dass sich eine gasdichte Verbindung zwischen der Wandung und der Abdeckung löst, wenn der Druck im Aufnahmeraum einen definierten Berstdruck übersteigt, wobei der Berstdruck einem Überdruck von 100 mbar, besonders bevorzugt 80 mbar und ganz besonders bevorzugt 60 mbar, insbesondere einem Überdruck von 50 mbar, entspricht.

Das Berstelement ist vorzugsweise zwischen einem umlaufenden Randbereich der Abdeckung und der Wandung des Behälters eingesetzt.

Eine gasdichte Verbindung des Randbereichs der Abdeckung mit dem Berstelement bzw. eine gasdichte Verbindung zwischen der Wandung und dem Berstelement lässt sich beispielsweise durch ein Verschweißen bzw. auch durch ein anderes stoffschlüssiges Verfahren, beispielsweise ein Verkleben, erzielen. Der Erfinder hat allerdings erkannt, dass es besonders vorteilhaft ist, wenn die Abdeckung, vorzugsweise der Randbereich der Abdeckung, und/oder die Wandung mit dem Berstelement verschraubt bzw. vernietet oder anderweitig kraftschlüssig verbunden ist. Vorzugsweise ist dabei vorgesehen, dass der umlaufende Randbereich der Abdeckung und/oder die Wandung Bohrungen aufweist, durch die jeweils eine Verschraubung mit dem Berstelement ermöglicht wird, vorzugsweise unter Zuhilfenahme weiterer Verbindungselemente, beispielsweise Flansche oder andere Profile.

Nachfolgend werden weitere Werte bzw. Wertbereiche für den Berstdruck genannt, die sich als vorteilhaft herausgestellt haben.

Von Vorteil ist es, wenn der Berstdruck einem Überdruck von wenigstens 30 mbar, vorzugsweise wenigstens 40 mbar, weiter bevorzugt wenigstens 50 mbar, ganz besonders bevorzugt wenigstens 60 mbar, insbesondere wenigstens 80 mbar, entspricht.

Diese Mindestwerte haben sich als besonders geeignet herausgestellt, um sicherzustellen, dass ein ausreichender Abstand zwischen dem gewünschten Betriebsdruck und dem Berstdruck, d. h. dem Überdruck, besteht, ab dem die gasdichte Verbindung zwischen der Wandung und der Abdeckung gelöst wird.

Von Vorteil ist es ferner, wenn der Berstdruck einem Überdruck von höchstens 200 mbar, vorzugsweise höchstens 150 mbar, weiter bevorzugt höchstens 120 mbar, noch weiter bevorzugt höchstens 100 mbar, ganz besonders bevorzugt 80 mbar, insbesondere höchstens 50 mbar, entspricht.

Die vorgenannten Werte haben sich als Höchstdrücke bewährt, um sicherzustellen, dass sich die gasdichte Verbindung zwischen der Abdeckung und der Wandung des Behälters bei einer außergewöhnlichen Gefährdungslage rechtzeitig löst, so dass das Gas in die Atmosphäre bzw. die Umgebung entweichen kann und somit Risiken durch eine, außergewöhnliche Umstände vorausgesetzt, Gasexplosion signifikant reduziert werden. Die Höchstwerte haben sich insbesondere in Kombination mit den vorgenannten Mindestwerten als besonders geeignet herausgestellt, wobei in diesem Fall die Werte (selbstverständlich) derart auszuwählen sind, dass der Mindestwert geringer ist als der Höchstwert. Ganz besonders vorteilhaft ist dabei ein Höchstwert von 50 mbar.

Von Vorteil ist es, wenn der Berstdruck einem Überdruck zwischen 30 und 150 mbar, vorzugsweise zwischen 30 und 100 mbar, weiter bevorzugt zwischen 30 und 80 mbar, noch weiter bevorzugt zwischen 30 und 60 mbar, ganz besonders bevorzugt zwischen 30 und 50 mbar insbesondere zwischen 40 und 50 mbar, entspricht.

Die vorgenannten Wertebereiche für den Berstdruck haben sich als besonders geeignet herausgestellt. In ganz besonderer Weise eignet es sich, einen Berstdruck einzustellen mit einem Wert zwischen 40 und 50 mbar. Somit ist der Abstand zu dem Betriebsdruck ausreichend, ferner ist sichergestellt, dass zu einem möglichst frühen und damit sicheren Zeitpunkt Gas in die Atmosphäre bzw. Umgebung abgegeben wird.

Erfindungsgemäß ist vorgesehen, dass das Berstelement beim Erreichen des definierten Berstdrucks die gasdichte Verbindung zwischen der Abdeckung und der Wandung löst.

Vorzugsweise ist das Berstelement als Berstring ausgebildet.

Das Berstelement, insbesondere in der Ausführung als Berstring, wie nachfolgend noch näher dargestellt wird, ist vorzugsweise derart gestaltet, dass die Abdeckung beim Erreichen des Berstdrucks vollständig von der Wandung gelöst wird, so dass großvolumig Gas aus dem Innenraum des Behälters austreten kann, nicht nur durch eine kleine Öffnung.

Der Berstring ist vorzugsweise derart gestaltet, dass die ganze Abdeckung wie ein Deckel von der Wandung abgehoben wird und sich dadurch ein entsprechender, vorzugsweise ringförmig umlaufender, Spalt zwischen der Abdeckung und der Wandung ergibt, wenn der Berstdruck einen definierten Wert übersteigt.

Der Berstring kann vorzugsweise als umlaufender Ring ausgebildet sein.

Vorzugsweise ist der Berstring vollständig ringförmig umlaufende zwischen der Abdeckung, vorzugsweise einem Randbereich der Abdeckung, und der Wandung des Behälters angeordnet.

Der Berstring kann auch teilringförmig ausgebildet sein. Ferner kann der Berstring auch durch ein oder mehrere Ringsegmente ausgebildet sein. Die Ringsegmente können dabei aneinander angrenzend oder auf Abstand zueinander angeordnet sein um einen Ring oder einen Teilring auszubilden. Die Verwendung des Begriffs Berstring im Rahmen der Erfindung schließt auch diese Gestaltung ein.

Dadurch, dass der gasdichte Behälter einen Berstring aufweist, kann definiert eingestellt werden, ab welchem Berstdruck sich die gasdichte Verbindung zwischen der Abdeckung und der Wandung lösen soll. Es kann dabei vorgesehen sein, dass der Berstring abgesprengt wird. Vorzugsweise ist der Berstring derart gestaltet, dass dieser ab dem definierten Berstdruck eine Verbindung vom Aufnahmeraum nach außen freigibt, vorzugsweise wenigstens teilweise ringförmig umlaufend um die Wandung (insbesondere symmetrisch), vorzugsweise vollständig ringförmig umlaufend, so dass der Überdruck bzw. das Gas aus dem Aufnahmeraum nach außen entweichen kann.

Von Vorteil ist es, wie bereits erwähnt, wenn der Berstring derart gestaltet ist, dass die Abdeckung beim Erreichen des Berstdrucks von der Wandung abgehoben wird. Es kann auch vorgesehen sein, dass die Verbindung zwischen dem Berstring und der Abdeckung und/oder zwischen dem Berstring und der Wandung teilweise oder vollständig aufgehoben wird, z. B. durch ein Anheben des Berstrings und/oder dessen Zerrstörung.

Erfindungsgemäß kann ferner vorgesehen sein, dass ein umlaufender Randbereich der Abdeckung an einer Unterseite mit einem unteren Flanschring und/oder an einer Oberseite mit einem oberen Flanschring versehen ist.

Die Verwendung eines unteren Flanschrings und/oder eines oberen Flanschrings hat sich als besonders geeignet herausgestellt, um die Abdeckung im Randbereich zu verstärken und den Randbereich stabil und gasdicht mit der Wandung zu verbinden.

In einer konstruktiven Ausgestaltung der Erfindung kann vorgesehen sein, dass der umlaufende Randbereich der Abdeckung zwischen dem unteren Flanschring und dem oberen Flanschring kraftschlüssig fixiert ist.

Es hat sich als besonders geeignet herausgestellt, wenn der umlaufende Randbereich der Abdeckung zwischen dem unteren Flanschring und dem oberen Flanschring kraftschlüssig eingeklemmt wird. Hierzu kann vorzugsweise eine Verschraubung vorgesehen sein. Dadurch lässt sich eine besonders stabile und auch wieder lösbare Verbindung zwischen den beiden Flanschringen und der Abdeckung herstellen.

Erfindungsgemäß kann vorgesehen sein, dass der untere Flanschring mit dem Berstring und der Berstring mit der Wandung des Behälters verbunden ist.

Der Erfinder hat erkannt, dass sich ein Lösen der Verbindung zwischen der Wandung und der Abdeckung besonders vorteilhaft dadurch erreichen lässt, dass der untere Flanschring mit dem Berstring und der Berstring mit der Wandung des Behälters verbunden ist. Durch eine geeignete Gestaltung oder Anordnung des Berstrings kann dabei erreicht werden, dass der Berstring die Verbindung zwischen der Wandung und dem unteren Flanschring und somit der Abdeckung löst. Dies kann beispielsweise dadurch erfolgen, dass der Berstring von der Wandung abgehoben wird oder die dort vorgesehene Verbindung bricht. Dies kann ergänzend oder alternativ auch dadurch erreicht werden, dass sich die Verbindung zwischen dem Berstring und dem unteren Flanschring löst, beispielsweise indem die Abdeckung angehoben wird oder die Verbindung zwischen dem Berstring und dem unteren Flanschring auf andere Weise gelöst wird, beispielsweise bricht.

Vorzugsweise wird die Verbindung zwischen dem unteren Flanschring und dem Berstring bzw. dem Berstring und der Wandung zumindest teilringförmig umlaufend, vorzugsweise vollständig ringförmig umlaufend, gelöst.

Die Abdeckung ist vorzugsweise derart gestaltet, dass diese die Oberseite der Wandung des Behälters abschließt und hierzu über das Berstelement vorzugsweise mit dem oberen Ende der umlaufenden Wandung des Behälters verbunden ist.

Von Vorteil ist es, wenn die Verbindung zwischen dem Berstring und der Wandung und/oder dem unteren Flanschring Langlöcher und/oder Schlitze aufweist, durch welche Befestigungselemente geführt sind, um den Berstring mit der Wandung und/oder dem unteren Flanschring zu verbinden.

Dadurch, dass die Verbindung zwischen dem Berstring und der Wandung und/oder zwischen dem Berstring und dem unteren Flanschring Langlöcher und/oder Schlitze aufweist, lässt sich eine besonders geeignete Verbindung zwischen dem Berstring und der Wandung und/oder dem Berstring und dem unteren Flanschring herstellen, die derart ausgebildet sein kann, dass sich die Verbindung löst, wenn der Druck in dem gasdichten Behälter den Berstdruck erreicht hat.

Es kann dabei vorgesehen sein, dass eine derartige Verbindung zwischen dem Berstring und der Wandung oder zwischen dem Berstring und dem unteren Flanschring oder auch an beiden Stellen ausgebildet ist.

Vorzugsweise wird jedes Befestigungselement der Verbindung durch ein Langloch bzw. einen Schlitz in einem der zu verbindenden Bauteile (Bersting oder Wandung bzw. Bersting oder unterer Flanschring) und eine herkömmliche Bohrung ggf. auch eine Sackbohrung in dem anderen zu verbindenden Bauteil (Wandung oder Berstring bzw. unterer Flanschring oder Berstring) geführt.

Es sei darauf hingewiesen, dass der Begriff "unterer Flanschring" nicht bedeutet, dass auch ein oberer Flanschring vorhanden sein muss.

Vorzugsweise sind die Langlöcher und/oder die Schlitze in dem Berstring ausgebildet.

Es kann jedoch auch vorgesehen sein, dass die Langlöcher und/oder die Schlitze in dem unteren Flanschring ausgebildet sind.

Es kann ferner vorgesehen sein, dass die Langlöcher und/oder die Schlitze in der Wandung ausgebildet sind.

Möglich ist es auch, dass sowohl im Berstring als auch in der Wandung und/oder dem unteren Flanschring Langlöcher und/oder Schlitze zur Durchführung der Befestigungselemente ausgebildet sind.

Es ist ferner auch möglich in dem Berstring und der Wandung und/oder dem unteren Flanschring sowohl Langlöcher und/oder Schlitze als auch herkömmliche Bohrungen vorzusehen, insbesondere wenn diese derart angeordnet sind, dass jedes Befestigungselement der Verbindung durch mindestens ein Langloch bzw. einen Schlitz geführt wird.

Erfindungsgemäß kann vorgesehen sein, dass die Verbindung zwischen dem Berstring und der Wandung und/oder dem unteren Flanschring derart beschaffen ist, dass die Verbindung in einem Betriebszustand des gasdichten Behälters gasdicht ist, jedoch beim Erreichen des Berstdrucks eine Relativbewegung zwischen dem Berstring und den Langlöchern und/oder den Schlitzen stattfindet, die dazu führt, dass sich die Abdeckung von der Wandung abhebt.

Dadurch, dass die Verbindung zwischen dem Berstring und der Wandung bzw. zwischen dem Berstring und dem unteren Flanschring über Langlöcher und Befestigungselemente, vorzugsweise Schrauben, erfolgt, kann in einfacher Weise erreicht werden, dass die Verbindung im normalen Betriebszustand des gasdichten Behälters, d. h. wenn der Betriebsdruck herrscht, gasdicht ist.

Die Verbindung kann dabei mit einfachen Maßnahmen jedoch derart ausgelegt werden, dass eine Relativbewegung zwischen den Befestigungselementen und den Langlöchern bzw. den Schlitzen der Verbindung stattfindet, wenn der Berstdruck erreicht bzw. überschritten wird. Das heißt, in diesem Fall wird die durch die Befestigungselemente zur Verfügung gestellte kraftschlüssige und/oder reibschlüssige Verbindung zwischen den zu verbindenden Bauteilen (Berstring und Wandung bzw. Berstring und unterem Flanschring) dadurch gelöst, dass die sich aus dem Berstdruck in dem gasdichten Behälter ergebenden Kräfte höher sind als die Haltekraft der kraftschlüssigen und/oder reibschlüssigen Verbindung zwischen den Bauteilen. Hieraus resultiert dann eine Relativbewegung zwischen den Befestigungselementen und den Langlöchern bzw. Schlitzen durch die sich folglich die Abdeckung von der Wandung abheben kann.

Wenn die Verbindung über Langlöcher und/oder Schlitze zwischen dem Berstring und der Wandung erfolgt, werden der Berstring und die Abdeckung (sowie die dazwischenliegenden weiteren Elemente) entsprechend angehoben. Vorzugsweise sind die Langlöcher und/oder die Schlitze dabei in dem Berstring ausgebildet.

Wenn die Verbindung über Langlöcher und/oder Schlitze zwischen dem Berstring und dem unteren Flanschring erfolgt, führt eine Relativbewegung zwischen den Befestigungselementen und den Langlöchern bzw. den Schlitzen dazu, dass der untere Flanschring und die Abdeckung (sowie die dazwischenliegenden weiteren Elemente) angehoben werden. Vorzugsweise sind die Langlöcher und/oder die Schlitze dabei in dem Berstring ausgebildet.

Der Gegenstand der Erfindung, nämlich dass zwischen der Abdeckung und der Wandung des Behälters ein Berstelement eingesetzt ist, welches beim Erreichen eines definierten Berstdrucks die gasdichte Verbindung zwischen der Abdeckung und der Wandung löst, gilt auch als erfüllt, wenn der untere Flanschring oder die Wandung über Langlöcher bzw. Schlitze verfügt. Auch in diesen Fällen wird im Sinne der Erfindung die gasdichte Verbindung zwischen der Abdeckung und der Wandung durch das eingesetzte Berstelement gelöst.

Von Vorteil ist es, wenn die Abdeckung aus vorzugsweise dünnen bzw. flachen Metallbahnen und/oder vorzugsweise dünnen bzw. flachen Metallplatten gebildet ist.

Im Stand der Technik wird eine derartige Abdeckung auch als "Membranabdeckung" bzw. "Membrandach" bezeichnet.

Die Abdeckung kann vorzugsweise aus dünnen bzw. flachen Metallbahnen gebildet sein, es ist jedoch auch möglich, die Abdeckung aus dünnen bzw. flachen Metallplatten oder einer Mischung hieraus auszubilden. Die Metallplatten können insbesondere auch ringförmig oder teilringförmig oder scheibenförmig ausgebildet sein.

Insofern nachfolgend auf eine Abdeckung aus Metallbahnen verwiesen wird, ist dies derart zu verstehen, dass alternativ die Abdeckung auch aus Metallplatten oder einer Mischung hieraus hergestellt sein kann.

Erfindungsgemäß kann ferner vorgesehen sein, dass wenigstens die Mehrzahl der flachen Metallbahnen und/oder der flachen Metallplatten der Abdeckung eine Stärke von 0,2 mm bis 5 mm, vorzugsweise 0,3 mm bis 2 mm, weiter bevorzugt 0,4 mm bis 1,5 mm, besonders bevorzugt 0,5 mm bis 1,2 mm und ganz besonders bevorzugt 0,6 mm bis 1,2 mm, insbesondere 1 mm, aufweist.

Die vorgenannten Werte haben sich als besonders geeignet herausgestellt, um eine leichte, flexible Abdeckung bereitzustellen. Eine derartige Abdeckung wird auch als Membranabdeckung bezeichnet.

Eine derartige Abdeckung erlaubt es, Behälter mit einem Durchmesser von bis zu 50 m frei zu überspannen. Die Abdeckung kann dabei, ohne dass im Aufnahmeraum Stützen oder Träger zur Abstützung der Abdeckung erforderlich sind, Schneelasten und starkem Wind widerstehen. Die sich aus einer derartigen Metallbahn ergebende Abdeckung eignet sich für gasdichte Behälter in besonderer Weise, insbesondere auch für Speicherbehälter für Gärreste.

Die Metallbahnen bzw. die Metallplatten sind vorzugsweise verschweißt, wobei sich eine Ausbildung der Abdeckung durch Metallbahnen bzw. Blechbahnen, die miteinander verschweißt sind, in besonderer Weise eignet.

Es hat sich gezeigt, dass sich eine Abdeckung, die in der Draufsicht betrachtet, rund bzw. kreisförmig ist, besonders eignet, um einen gasdichten Behälter abzuschließen. Entsprechend ist es besonders geeignet, wenn die Wandung des gasdichten Behälters ebenfalls kreisförmig ausgebildet ist bzw. wenn die Wandung eine zylinderförmige Wandung ist.

Es hat sich ferner als besonders geeignet herausgestellt, wenn die Abdeckung als flaches Dach ausgebildet ist.

Von Vorteil ist es, wenn die Abdeckung, nachdem die Metallbahnen bzw. die Metallplatten miteinander verschweißt sind, auf eine Walze oder Rolle aufgewickelt und in einem derartigen aufgewickelten Zustand auf die Baustelle transportiert wird. Die Abdeckung kann dann auf der Baustelle an der Oberseite der Wandung wieder ausgerollt und in der bereits beschriebenen Art und Weise mit der Oberseite der Wandung gasdicht verbunden werden.

Eine Abdeckung aus den vorgenannten Metallbahnen bzw. Metallplatten, insbesondere in einer Ausgestaltung aus Edelstahl, hat den Vorteil, dass die Abdeckung korrosionsbeständig ist, diffusionsdicht für Gas ist und unempfindlich gegen UV-Strahlung ist. Die Abdeckung kann für beliebige Behälter, vorzugsweise für Metallbehälter, insbesondere Stahlbehälter, als auch für Betonbehälter sowie für Metallbetonbehälter, insbesondere für Stahlbetonbehälter, eingesetzt werden. Die Wandung auf deren oberes Ende die Abdeckung aufgebracht wird, kann aus einem beliebigen Material, vorzugsweise Metall, insbesondere Stahl, Beton, Metallbeton, insbesondere Stahlbeton, gebildet sein, bzw. eines der Materialien als Hauptbestandteil aufweisen.

Die Abdeckung kann vorzugsweise komplett im Werk vorgefertigt und nachträglich auf der Baustelle montiert werden. Insbesondere wenn in einer bevorzugten Ausführungsform eine kraftschlüssige Verbindung zwischen der Wandung und der Abdeckung vorgesehen ist, kann auf Schweißarbeiten vor Ort verzichtet werden.

In einer besonders bevorzugten Ausgestaltung kann vorgesehen sein, dass die Dacheinrichtung eine oberhalb der Abdeckung angeordnete tragende Konstruktion und eine Aufhängeeinrichtung aufweist, wobei die Aufhängeeinrichtung an der Abdeckung und an der tragenden Konstruktion festgelegt ist und die Aufhängeeinrichtung ausgebildet ist, um die Abdeckung in Richtung auf die tragende Konstruktion nach oben zu verspannen.

Es sei darauf hingewiesen, dass die Angaben "oben" bzw. "unten" bzw. "oberhalb" bzw. "unterhalb" sich auf einen Behälter beziehen, der korrekt auf einer Unterlage, insbesondere einen Betonsockel oder dergleichen, montiert ist.

Der Erfinder hat erkannt, dass dadurch, dass die Abdeckung verspannt wird, eine unerwünschte Bewegung, insbesondere ein Flattern der Abdeckung, vermieden wird. Insbesondere wenn der gasdichte Behälter als Biogasreaktor bzw. als Fermenter ausgebildet ist, konnte es bei den vorbekannten Behältern zu einem ungewünschten "Flattern" der aus den dünnen Metallbahnen bzw. Metallplatten gebildeten Abdeckung kommen.

Die vorteilhafte Ausgestaltung ermöglicht es nunmehr, die aus den Metallbahnen bzw. Metallplatten gebildete Abdeckung für gasdichte Behälter zu verwenden, ohne dass die bislang auftretenden Nachteile, nämlich das "Flattern", in Kauf genommen werden müssen.

Die erfindungsgemäße Lösung verbessert die Konstruktion und ermöglicht es insbesondere, eine vergleichsweise dünne Abdeckung einzusetzen.

Von Vorteil ist es, wenn die Aufhängeeinrichtung die Abdeckung derart nach oben verspannt, dass die Abdeckung konvex nach oben gewölbt ist.

Grundsätzlich wäre es auch möglich, dass die Aufhängeeinrichtung derart ausgebildet ist, dass diese die Abdeckung nach unten verspannt, d. h. eine Druckkraft auf die Abdeckung ausübt. Es hat sich jedoch als geeigneter herausgestellt, wenn die Aufhängeeinrichtung die Abdeckung nach oben zieht bzw. nach oben verspannt.

Von Vorteil ist es, wenn die Abdeckung freitragend ausgebildet ist.

Der Erfinder hat erkannt, dass sich eine Abdeckung bei dem erfindungsgemäßen gasdichten Behälter besonders vorteilhaft dadurch realisieren lässt, dass diese freitragend ausgebildet ist. Hierunter ist im Rahmen der Erfindung zu verstehen, dass die Abdeckung nur von der Wandung getragen wird und keine zusätzlichen Stützen, insbesondere keine mittleren Mittelstützen, vorgesehen sind. Im Rahmen der erfindungsgemäßen Lösung ist jedoch vorgesehen, dass die Abdeckung von der Aufhängeeinrichtung nach oben verspannt wird. Im Sinne der Erfindung gilt die Abdeckung dabei dennoch als freitragend, insbesondere da diese zwischen den Randbereichen keine zusätzlichen tragenden Stützen oder dergleichen aufweist.

Die freitragende, leichte Abdeckung aus Edelstahl erlaubt es, sowohl bestehende gasdichte Behälter mit der Abdeckung nachzurüsten, als auch neue Behälter entsprechend herzustellen.

Von Vorteil ist es, wenn die tragende Konstruktion als Kegeldach und/oder als Skelettkonstruktion ausgebildet ist.

Die Ausbildung der tragenden Konstruktion, an der dann auch die Aufhängeeinrichtung festgelegt wird, als Kegeldach und/oder als Skelettkonstruktion hat sich als besonders geeignet herausgestellt. Dadurch wird eine Konstruktion gewählt, die einerseits hohe Kräfte aufnehmen kann und andererseits durch bekannte Maßnahmen zuverlässig und prozesssicher realisiert werden kann.

Vorgesehen sein kann insbesondere, dass die tragende Konstruktion des Kegeldachs bzw. die Skelettkonstruktion an der Wandung, vorzugsweise auf der Oberseite der Wandung, entsprechend festgelegt ist, so dass die Kräfte direkt in die Wandung eingeleitet werden können und von dort nach unten auf den Boden, auf dem die Wandung steht, abgeleitet werden.

Von Vorteil ist es, wenn die tragende Konstruktion selbst nicht gasdicht ausgebildet ist.

Grundsätzlich könnte die tragende Konstruktion, das heißt beispielsweise das Kegeldach und/oder die Skelettkonstruktion, ebenfalls gasdicht ausgebildet sein. Dies erhöht jedoch die Baukosten signifikant und ist aufgrund der gasdicht ausgebildeten Abdeckung vorliegend nicht notwendig.

Alternativ zu der Ausgestaltung der tragenden Konstruktion als Kegeldach und/oder als Skelettkonstruktion kann erfindungsgemäß auch vorgesehen sein, dass die tragende Konstruktion durch horizontal verlaufende Balken gebildet ist. Die Balken sind dabei vorzugsweise an der Oberseite der Wandung befestigt bzw. dort aufgelegt.

Die Ausbildung einer tragenden Konstruktion als Kegeldach und/oder als Skelettkonstruktion hat sich jedoch als vorteilhaft herausgestellt, insbesondere um die Aufhängeeinrichtung oberhalb der Abdeckung stabil festlegen zu können.

Von Vorteil ist es dabei auch, wenn die tragende Konstruktion über eine begehbare Plattform verfügt, die vorzugsweise auch oberhalb der Aufhängeeinrichtung angeordnet ist. Eine Bedienung der Aufhängeeinrichtung kann dadurch erleichtert werden.

Erfindungsgemäß kann ferner vorgesehen sein, dass die Aufhängeeinrichtung mittig oberhalb der Abdeckung, vorzugsweise koaxial zu der vertikal verlaufenden Mittelachse des Behälters angeordnet ist.

Eine Anordnung der Aufhängeeinrichtung mittig oberhalb der Abdeckung hat sich als besonders geeignet herausgestellt, um die Abdeckung nach oben in Richtung auf die tragende Konstruktion verspannen zu können. Es hat sich insbesondere als vorteilhaft herausgestellt, wenn die Abdeckung primär in einem mittleren Bereich nach oben verspannt wird. Eine besonders symmetrische Anordnung der Aufhängeeinrichtung bewährt sich dabei, um die Kräfte in die Abdeckung möglichst gleichmäßig einzuleiten.

Die Aufhängeeinrichtung kann unmittelbar an der Abdeckung festgelegt sein. Von Vorteil ist es jedoch, wenn die Aufhängeeinrichtung an einem Flanschring oder einem Ringsegment oder einem Befestigungselement oder einer Befestigungsplatte festgelegt ist, welche selbst wiederum an der Abdeckung festgelegt ist. In besonders vorteilhafter Weise eignet sich ein Flanschring mit einem Durchmesser, der in etwa dem Durchmesser der Aufhängeeinrichtung entspricht.

Von Vorteil ist es, wenn die Aufhängeeinrichtung an Befestigungspunkten der Abdeckung festgelegt ist, und wenn der Abstand der Befestigungspunkte von dem Mittelpunkt der Abdeckung weniger als 70 %, vorzugsweise weniger als 60 %, weiter bevorzugt weniger als 50 %, besonders bevorzugt weniger als 40 % und ganz besonders bevorzugt weniger als 30 % des Abstands des Mittelpunkts der Abdeckung zum Rand der Abdeckung beträgt.

Die vorgenannte Festlegung der Aufhängeeinrichtung an der Abdeckung hat sich als besonders geeignet herausgestellt, um die Abdeckung nach oben zu verspannen. Besonders zu bevorzugen ist es dabei, wenn die Aufhängeeinrichtung symmetrisch bzw. mittig um einen Mittelpunkt der Abdeckung herum angeordnet ist.

Die Befestigungspunkte der Abdeckung, an denen die Aufhängeeinrichtung festgelegt ist, können dabei auch an einem Befestigungsring, einem Flanschring einer Befestigungsplatte oder einem Ringsegment, welcher an der Abdeckung festgelegt ist, ausgebildet sein.

Erfindungsgemäß kann ferner vorgesehen sein, dass die Aufhängeeinrichtung eine Mehrzahl von Ketten aufweist. Die Ketten können dabei unmittelbar oder über eines der vorgenannten Verbindungselemente an der Abdeckung festgelegt sein, um die Abdeckung in Richtung auf die tragende Konstruktion nach oben zu spannen.

Es hat sich als besonders geeignet herausgestellt, wenn die Aufhängeeinrichtung eine Mehrzahl an Ketten aufweist, die unmittelbar oder über ein Verbindungselement, beispielsweise den vorgenannten Flanschring, an der Abdeckung festgelegt ist. Vorgesehen sein kann dabei auch, dass die Aufhängeeinrichtung ausgebildet ist, um die Abdeckung innerhalb eines definierten Hubs anzuheben und abzusenken. Hierzu kann beispielsweise eine Einstelleinrichtung, beispielsweise in Gestalt von Gewindestangen, vorgesehen sein, die es ermöglichen, die Aufhängeeinrichtung zu lösen oder nachzuziehen, um den Hub bzw. die Verspannung der Abdeckung in Richtung auf die tragende Konstruktion einzustellen.

Es eignet sich dabei in besonderer Weise, wenn die Gewindestangen jeweils mit einer Kette oder einem anderen Aufhängeglied der Aufhängeeinrichtung zusammenwirken.

Erfindungsgemäß kann vorgesehen sein, dass im Aufnahmeraum ein Rührwerk angeordnet ist.

Es hat sich als besonders geeignet herausgestellt, wenn das Rührwerk von oben oder von der Seite (wie beispielsweise in der DE 199 54 904 A1 dargestellt) zugeführt wird.

Grundsätzlich kann im Aufnahmeraum auch mehr als nur ein Rührwerk vorgesehen sein.

Insofern vorgesehen ist, das Rührwerk von oben zuzuführen, ist es von Vorteil, wenn die Abdeckung eine Rührwerksdurchführung aufweist, welche vorzugsweise koaxial zu der vertikalen Mittelachse des Behälters verläuft.

Die Rührwerksdurchführung kann dabei mit Dichtungen versehen sein, derart dass die Rührwerksdurchführung gasdicht mit dem Rührwerk abschließt.

Das Rührwerk kann in grundsätzlich bekannter Art und Weise gestaltet sein.

Es kann sich in besonderer Weise eignen, wenn sich das Rührwerk bzw. die Welle des Rührwerks von oben über mindestens 50 % der Höhe des Aufnahmeraums erstreckt. Von Vorteil kann es ferner sein, wenn in regelmäßigen oder unregelmäßigen Abständen an der Welle des Rührwerks Flügel vorgesehen sind, durch die das in den Aufnahmeraum eingebrachte Material, insbesondere eine organische Substanz, bewegt werden kann. Vorzugsweise sind die Flügel anklappbar oder entsprechend flexibel, so dass der Radius der Flügel zur Montage oder Demontage des Rührwerks reduziert werden kann.

Von Vorteil ist es, wenn der gasdichte Behälter über eine Heizeinrichtung in der Wandung verfügt.

Insbesondere bei einem Einsatz des gasdichten Behälters als Bioreaktor bzw. Fermenter kann es ferner geeignet sein, wenn die Wandung isoliert ist. Vorzugsweise ist auch die Dacheinrichtung isoliert.

Vorzugsweise ist der gasdichte Behälter, insbesondere der Aufnahmeraum des gasdichten Behälters, über einen Gasauslass mit einem Gasspeicher verbunden.

Der Aufnahmeraum kann ferner über weitere Elemente verfügen, beispielsweise derart, wie diese in der DE 199 54 904 A1 dargestellt sind. Erwähnt sei beispielsweise ein Füllstutzen, um frische organische Substanzen in den Aufnahmeraum, der in diesem Fall auch als Gärraum bezeichnet werden kann, einzufüllen.

Der erfindungsgemäße gasdichte Behälter kann insbesondere in einer Ausgestaltung als Fermenter bzw. Biogasreaktor auch als Kombination von Faulbehälter und Gasspeicher ausgebildet sein. Der Gasspeicher kann in diesem Fall oberhalb der Abdeckung positioniert sein, vorzugsweise derart, dass sich dieser innerhalb der tragenden Konstruktion, insbesondere wenn diese als Kegeldach und/oder als Skelettkonstruktion ausgebildet ist, befindet.

Die erfindungsgemäße Lösung eignet sich jedoch in besonderer Weise, wenn ein Gasspeicher außerhalb des gasdichten Behälters positioniert und über eine Verrohrung mit einem Gasauslass des gasdichten Behälters verbunden ist.

Erfindungsgemäß kann vorgesehen sein, dass die Abdeckung wenigstens einen äußeren Abdeckungsring mit einer inneren Öffnung aufweist und die Abdeckung ferner eine innere Abdeckscheibe aufweist, welche die innere Öffnung des äußeren Abdeckungsrings gasdicht verschließt.

Die vorgenannte Lösung stellt eine besonders geeignete Konstruktion dar, um eine Zugänglichkeit in den Aufnahmeraum sicherzustellen, ohne dass die gesamte Abdeckung wieder entfernt werden muss. Somit kann zum Beispiel ein sogenanntes "Mannloch" bereitgestellt werden. Um in den Aufnahmeraum zu gelangen, beispielsweise um ein Rührwerk einzubringen oder zu entfernen, ist es ausreichend, wenn die innere Abdeckscheibe demontiert wird. Dadurch wird die innere Öffnung des äußeren Abdeckungsrings freigegeben, die vorzugsweise derart dimensioniert ist, dass ein Rührwerk in den Aufnahmeraum eingebracht und von dort wieder entfernt werden kann.

Die innere Abdeckscheibe kann dabei eine Rührwerksdurchführung für das Rührwerk aufweisen.

Insofern das Rührwerk seitlich in den Aufnahmeraum eingeführt wird, kann auf eine Rührwerksdurchführung in der inneren Abdeckscheibe verzichtet werden.

In einer besonders vorteilhaften Ausgestaltung der Erfindung kann vorgesehen sein, dass die innere Abdeckscheibe Bohrungen aufweist, durch die Schrauben steckbar sind, um die Abdeckscheibe mit dem äußeren Abdeckungsring zu verschrauben. Dabei kann vorgesehen sein, dass an der dem Aufnahmeraum zugewandten inneren Seite der Abdeckscheibe die Schraubenköpfe nach dem Durchstecken jeweils mit der Abdeckscheibe verschweißt sind, um diese verliersicher zu halten.

Erfindungsgemäß kann vorgesehen sein, dass der Aufnahmeraum ein Volumen von 100 bis 50.000 m³, vorzugsweise von 1.000 bis 10.000 m³, besonders bevorzugt von 2.000 bis 8.000 m³ aufweist.

Der Erfinder hat erkannt, dass sich die erfindungsgemäße Lösung insbesondere für Aufnahmeräume mit den vorgenannten Volumina eignet.

Die Höhe des gasdichten Behälters kann vorzugsweise 2 bis 50 m und die Breite bzw. der Durchmesser vorzugsweise 4 bis 50 m betragen.

Der gasdichte Behälter kann insbesondere für Klärgas, Deponiegas oder Biogas vorgesehen sein.

Die vorliegende Erfindung betrifft auch eine Vorrichtung zur Gewinnung eines gasförmigen Energieträgers aus organischen Substanzen, insbesondere einen Biogasreaktor, aufweisend einen gasdichten Behälter mit einem oder mehreren der vorstehend dargestellten Merkmalen.

Mögliche Ausgestaltungen und auch die Vorteile einer derartigen Vorrichtung zur Gewinnung eines gasförmigen Energieträgers, aufweisend einen gasdichten Behälter, ergeben sich aus den vorhergehenden und auch aus den nachfolgenden Ausführungen entsprechend.

Die Figuren zeigen jeweils bevorzugte Ausführungsbeispiele, in denen einzelne Merkmale der vorliegenden Erfindung in Kombination miteinander dargestellt sind. Die Merkmale eines Ausführungsbeispiels sind auch losgelöst von den anderen Merkmalen des gleichen Ausführungsbeispiels umsetzbar und können dementsprechend von einem Fachmann zu weiteren sinnvollen Kombinationen mit Merkmalen anderer Ausführungsbeispiele verbunden werden.

Es zeigen schematisch:
- Figur 1: einen Längsschnitt durch einen erfindungsgemäßen gasdichten Behälter;
- Figur 2: eine Detaildarstellung eines Ausschnitts einer Dacheinrichtung des erfindungsgemäßen gasdichten Behälters;
- Figur 3: eine Draufsicht auf eine Abdeckung des gasdichten Behälters in einer ersten Ausführungsform;
- Figur 4: eine Draufsicht auf eine Abdeckung des gasdichten Behälters in einer zweiten Ausführungsform;
- Figur 5: eine Darstellung einer möglichen Verbindung zwischen einer Abdeckung und einer Wandung unter Verwendung eines erfindungsgemäßen Berstelements in einer Ausführung als Berstring; und
- Figur 6: eine vergrößerte Darstellung des Bereichs VI der Figur 5.

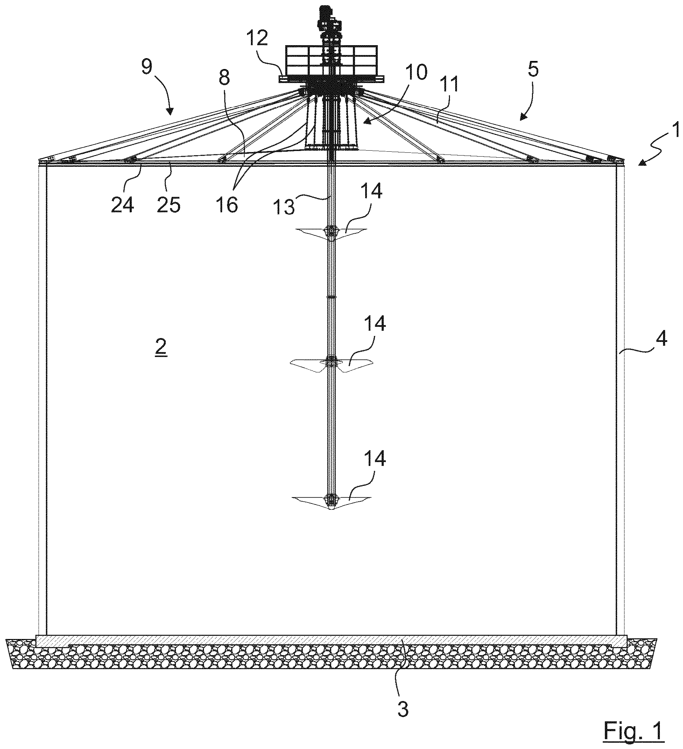

Die Ausführungsbeispiele nach den Figuren 1 bis 6 zeigen einen gasdichten Behälter 1, der sich grundsätzlich für verschiedene Anwendungen eignen kann. Der gasdichte Behälter 1 kann insbesondere zur Lagerung von festen, flüssigen und gasförmigen Medien, insbesondere auch zur Lagerung von organischen Substanzen, verwendet werden.

Der gasdichte Behälter 1 ist im Ausführungsbeispiel als Großraumbehälter ausgeführt.

Im Ausführungsbeispiel ist vorgesehen, dass der gasdichte Behälter 1 als Fermenter bzw. als sogenannter Biogasreaktor ausgebildet ist. Der gasdichte Behälter 1 kann dabei Teil einer Vorrichtung zur Gewinnung eines gasförmigen Energieträgers aus organischen Substanzen sein. Die Vorrichtung kann zusätzlich weitere Einrichtungen, beispielsweise einen Gasspeicher, aufweisen. Hierzu kann vorzugsweise wenigstens ein Gasauslass vorgesehen sein, vorzugsweise im oberen Bereich des Behälters 1, von dem das Gas in den Gasspeicher strömt.

Im nachfolgenden Ausführungsbeispiel ist der gasdichte Behälter 1 nur soweit dargestellt, wie dies zur Erläuterung der vorliegenden Erfindung wesentlich ist.

In einer bevorzugten Ausführungsform, bei der der gasdichte Behälter 1 als Fermenter ausgebildet ist, kann insbesondere vorgesehen sein, dass dieser über weitere Elemente verfügt, insbesondere über einen Gasauslass, Heizeinrichtungen, Zuführöffnungen, um organische Substanzen einzubringen, und dergleichen, so wie dies beispielsweise aus der DE 199 54 904 A1 oder der DE 197 56 485 A bekannt ist, deren jeweiliger Inhalt durch Bezugnahme vollständig in die vorliegende Beschreibung integriert sei.

Insbesondere auch hinsichtlich des Aufbaus der nachfolgend noch beschriebenen Wandung 4 des Behälters 1 können Merkmale aus den beiden vorgenannten Dokumenten eingesetzt werden.

Es sei darauf hingewiesen, dass der gasdichte Behälter 1 im Ausführungsbeispiel zwar vorzugsweise als Fermenter ausgebildet, das Ausführungsbeispiel jedoch hierauf nicht beschränkt zu verstehen ist.

Wie aus den Ausführungsbeispielen ersichtlich ist, weist der gasdichte Behälter 1 einen Aufnahmeraum 2, einen Boden 3, eine umlaufende Wandung 4 und eine Dacheinrichtung 5 auf.

Im Ausführungsbeispiel wird der Aufnahmeraum 2 von dem Boden 3, der Wandung 4 und der Dacheinrichtung 5 gasdicht umschlossen. Der Aufnahmeraum 2 kann dabei insbesondere als Gärraum, insbesondere zur Aufnahme von organischen Substanzen, ausgebildet sein.

Die Wandung 4 kann aus einem beliebigen Material, vorzugsweise aus Metall, Beton oder Metallbeton, insbesondere Stahl oder Stahlbeton, ausgebildet sein bzw. die vorgenannten Materialien als Hauptbestandteil aufweisen. Vorzugsweise ist die Wandung 4 durch Blechstreifen gebildet, die miteinander verschweißt werden. Vorgesehen sein kann auch ein Doppelfaltsystem, so wie dies von der Lipp GmbH, Tannhausen eingesetzt wird, um die Wandung aus Blechstreifen auszubilden.

Der Boden 3 kann beliebig ausgebildet sein, vorzugsweise ist der Boden 3 durch Stahlplatten ausgebildet und/oder betoniert.

Der Aufnahmeraum 2 kann im Ausführungsbeispiel ein Volumen von 100 bis 50.000 m³, vorzugsweise von 1.000 bis 10.000 m³, besonders bevorzugt von 2.000 bis 8.000 m³, aufweisen.

Ferner eignet es sich für die Erfindung in besonderer Weise, wenn der gasdichte Behälter 1 eine Höhe von 2 bis 50 m und eine Breite von 4 bis 50 m aufweist.

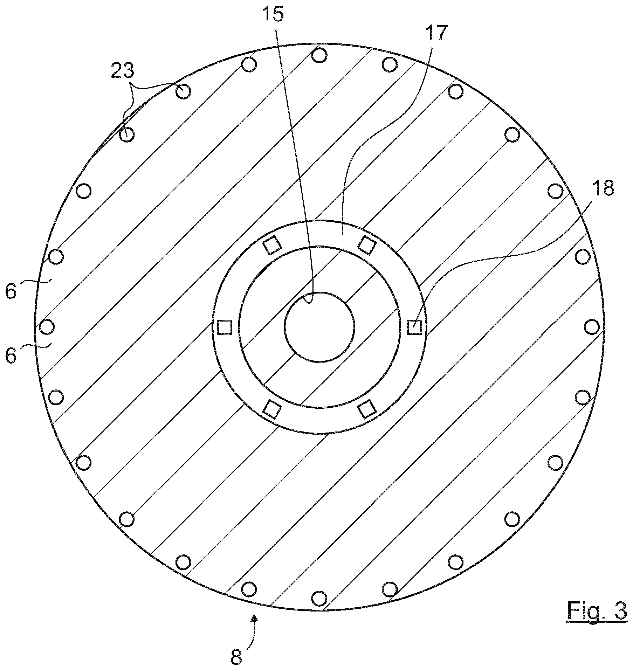

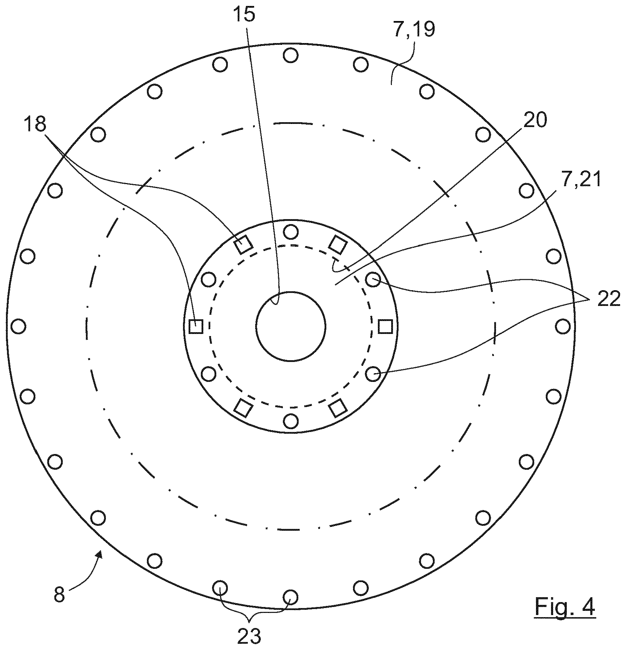

Die Dachkonstruktion 5 weist, wie aus den Ausführungsbeispielen ersichtlich ist, vorzugsweise eine gasdichte, aus flachen Metallbahnen 6 und/oder Metallplatten 7 gebildete Abdeckung 8 auf. Grundsätzlich ist es möglich, die Abdeckung 8 aus Metallbahnen 6 oder Metallplatten 7 oder einer Mischung aus Metallbahnen 6 und Metallplatten 7 herzustellen. Im Ausführungsbeispiel ist in der Figur 3 eine Draufsicht auf eine Abdeckung 8 dargestellt, die aus Metallbahnen 6 gebildet ist, während in der Figur 4 eine Draufsicht auf eine Abdeckung 8 gezeigt ist, die aus Metallplatten 7 gebildet ist.

Insofern im Ausführungsbeispiel auf eine Ausbildung der Abdeckung 8 aus Metallbahnen 6 bzw. Metallplatten 7 verwiesen wird, ist dies derart zu verstehen, dass alternativ auch die jeweils andere Gestaltung gewählt werden kann.

In dem in Figur 4 dargestellten Ausführungsbeispiel ist vorgesehen, dass die Metallplatten 7 als Metallringe, wie nachfolgend noch näher dargestellt wird, ausgebildet sind.

Die Abdeckung 8 ist an der Oberseite der Wandung 4 festgelegt und gasdicht mit der Wandung 4 verbunden.

Im Ausführungsbeispiel ist dabei vorgesehen, dass die Abdeckung 8 an ihrem umlaufenden Randbereich mit der Wandung 4 gasdicht verbunden ist.

Im Ausführungsbeispiel ist ferner vorgesehen, dass wenigstens die Mehrzahl der flachen Metallbahnen 6 und/oder der flachen Metallplatten 7 der Abdeckung 8 eine Stärke von 0,2 mm bis 5 mm, vorzugsweise 0,3 mm bis 2 mm, weiter bevorzugt 0,4 mm bis 1,5 mm, besonders bevorzugt 0,5 mm bis 1,2 mm und ganz besonders bevorzugt 0,6 mm bis 1,2 mm, insbesondere 1 mm, aufweist.

Dadurch, dass die Metallbahnen 6 als dünne und flache Metallbahnen ausgebildet sind, ergibt sich eine leichte Abdeckung, eine sogenannte Membrankonstruktion, die es erlaubt, auch große Durchmesser frei bzw. freitragend mit der Abdeckung 8 zu überspannen, das heißt, dass keine weiteren Stützen unterhalb der Abdeckung 8 erforderlich sind. Trotzdem kann die Abdeckung 8 hohe Lasten aufnehmen.

Ein besonderer Vorteil einer derart gebildeten Abdeckung 8 besteht auch darin, dass diese auf Rollen oder Walzen aufgewickelt und dann vor Ort, das heißt auf der Baustelle, wieder abgewickelt und mit der Wandung 4 verbunden werden kann. Die Wandung 4 kann dabei insbesondere aus den vorstehend bereits genannten Materialien (Metall, insbesondere Stahl, Beton, Metallbeton, insbesondere Stahlbeton) aufgebaut bzw. gebildet sein.

Die flachen Metallbahnen 6 bzw. die Metallplatten 7 sind im Ausführungsbeispiel vorzugsweise aus Edelstahl ausgebildet.

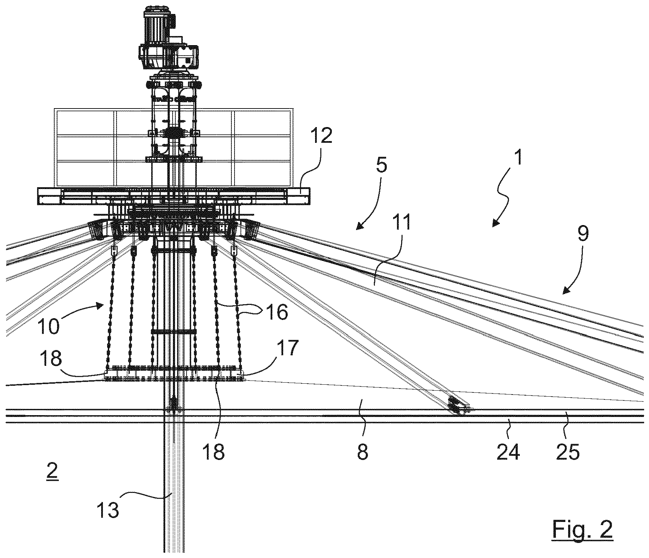

Wie aus den Figuren 1 und 2 ersichtlich ist, weist die Dacheinrichtung 5 oberhalb der Abdeckung 8 vorzugsweise eine tragende Konstruktion 9 und eine Aufhängeeinrichtung 10 auf. Die Aufhängeeinrichtung 10 ist dabei an der Abdeckung 8 und an der tragenden Konstruktion 9 festgelegt. Die Aufhängeeinrichtung 10 ist ausgebildet, um die Abdeckung 8 in Richtung auf die tragende Konstruktion 9 nach oben zu verspannen. Wie aus den Figuren 1 und 2 ersichtlich ist, wird die Abdeckung 8 von der Aufhängeeinrichtung 10 derart nach oben verspannt, dass die Abdeckung 8 konvex nach oben, das heißt in Richtung auf die tragende Konstruktion 9, gewölbt ist.

Die tragende Konstruktion 9 ist im Ausführungsbeispiel als Kegeldach bzw. als Skelettkonstruktion ausgebildet. Die tragende Konstruktion 9 weist hierzu beispielsweise Sparren 11 auf, die vorzugsweise an der Oberseite der Wandung 4 festgelegt sind, damit die Gewichtskräfte in die Wandung 4 eingeleitet werden können.

Im Ausführungsbeispiel ist die tragenden Konstruktion 9 vorzugsweise als ein tragendes Element des gasdichten Behälters 1 ausgebildet, das heißt, dass die tragende Konstruktion 9 der Dacheinrichtung 5 Gewichtskräfte aufnehmen kann, insbesondere auch derart, dass die Abdeckung 8 durch die Aufhängeeinrichtung 10 nach oben verspannt werden kann.

Wie aus den Figuren 1 und 2 ersichtlich ist, weist die tragende Konstruktion 9 optional eine Plattform 12 auf. Die Plattform 12 ist dabei vorzugsweise im Bereich der Spitze bzw. des oberen Endes des gasdichten Behälters 1 bzw. der Spitze der Dacheinrichtung 5 angeordnet. Vorzugsweise befindet sich die Plattform 12 in der Mitte der Dacheinrichtung 5 bezogen auf die horizontale Erstreckung der Dacheinrichtung 5. Die Plattform 12 kann insbesondere dazu dienen, die Aufhängeeinrichtung 10 zu warten und zu bedienen. Die Plattform 12 kann zusätzlich auch dazu verwendet werden, ein - optional an dieser Stelle angeordnetes - Rührwerk 13 zu montieren, zu demontieren, zu warten und zu bedienen.

Das Rührwerk 13 wird im Ausführungsbeispiel ebenfalls von der tragenden Konstruktion 9 getragen. Rührwerke 13 sind für gasdichte Behälter, insbesondere für Fermenter, grundsätzlich aus dem Stand der Technik bekannt. Hierzu wird beispielsweise auf die DE 199 54 904 A1 verwiesen, welche ein seitlich in den Aufnahmeraum 2 eingebrachtes Rührwerk offenbart.

Auch im Rahmen des vorliegenden Ausführungsbeispiels kann alternativ oder ergänzend zu einem Rührwerk 13, so wie dies in den Figuren 1 und 2 dargestellt ist, ein Rührwerk auch seitlich in den Aufnahmeraum 2 eingeführt werden.

Im Ausführungsbeispiel ist vorgesehen, dass sich das Rührwerk 13 von der Dacheinrichtung 5, im Ausführungsbeispiel von oberhalb der Plattform 12, in den Aufnahmeraum 2 erstreckt. Das Rührwerk 13 verläuft dabei vorzugsweise entlang der sich in vertikaler Richtung erstreckenden Mittelachse des Behälters 1, vorzugsweise koaxial zu der vertikal verlaufenden Mittelachse des Behälters 1.

Das Rührwerk 13 kann in bekannter und nicht näher bezeichneter Weise einen Motor aufweisen. Ferner ist im Ausführungsbeispiel vorgesehen, dass das Rührwerk 13 einen oder mehrere Flügel 14 aufweist, um das in den Aufnahmeraum 2 eingebrachte Material, vorzugsweise eine organische Substanz, zu verrühren bzw. in Bewegung zu versetzen. Die Flügel 14 sind im Ausführungsbeispiel vorzugsweise anklappbar, so dass das Rührwerk durch entsprechende Öffnungen in der Dacheinrichtung 5 entnommen werden kann.

Im Ausführungsbeispiel ist vorgesehen, dass die Abdeckung 8 eine Rührwerksdurchführung 15 aufweist, welche vorzugsweise koaxial zu der vertikalen Mittelachse des Behälters 1 verläuft. Die Rührwerksdurchführung 15 ist beispielhaft in den Figuren 3 und 4 dargestellt. Die Rührwerksdurchführung 15 verfügt über nicht näher dargestellte Dichtungen, damit die Rührwerksdurchführung 15 gasdicht mit dem Rührwerk 13 bzw. dessen Welle abschließt.

Im Ausführungsbeispiel ist vorgesehen, dass die tragende Konstruktion 9 selbst nicht gasdicht ausgebildet ist. Grundsätzlich wäre dies jedoch möglich.

Wie aus den Ausführungsbeispielen ersichtlich ist, ist die Aufhängeeinrichtung 10 mittig oberhalb der Abdeckung 8, vorzugsweise koaxial zu der vertikal verlaufenden Mittelachse des Behälters 1 angeordnet. Die Aufhängeeinrichtung 10 ist ausgebildet, um die Abdeckung 8 innerhalb eines definierten Hubs anzuheben und gegebenenfalls wieder abzusenken.

Die Aufhängeeinrichtung 10 ist insbesondere vorgesehen, um die Abdeckung 8 derart nach oben zu verspannen, dass ein Flattern der Abdeckung 8, beispielsweise aufgrund von schwankenden Drücken im Aufnahmeraum 2, vermieden wird.

Grundsätzlich kann die Aufhängeeinrichtung 10 beliebig aufgebaut sein und an beliebigen Stellen der Abdeckung 8 angreifen. Das Ausführungsbeispiel ist nicht auf eine spezifische Konstruktion beschränkt zu verstehen. Es hat sich jedoch als besonders geeignet herausgestellt, wenn die Aufhängeeinrichtung 10 an Befestigungspunkten 18 der Abdeckung 8 festgelegt ist, und der Abstand der Befestigungspunkte 18 von dem Mittelpunkt der Abdeckung 8 weniger als 70 %, vorzugsweise weniger als 60 %, weiter bevorzugt weniger als 50 %, besonders bevorzugt weniger als 40 % und ganz besonders bevorzugt weniger als 30 % des Abstands des Mittelpunkts der Abdeckung 8 zum Rand der Abdeckung 8 beträgt.

Dadurch, dass die Aufhängeeinrichtung 10 vorzugsweise in einem inneren Kreis der Abdeckung 8 angreift und die Abdeckung nach oben verspannt, ergibt sich eine besonders geeignete Verspannung der Abdeckung 8, mit der ein "Flattern" der Abdeckung 8 zuverlässig vermieden wird.

Die Aufhängeeinrichtung 10 kann über nicht näher dargestellte Einrichtungen verfügen, um den Hub, mit dem die Abdeckung 8 angehoben wird, zu verändern. Hierzu können beispielsweise Gewindestangen vorgesehen sein, die in geeigneter Weise verdreht werden, um die vertikale Länge der Aufhängeeinrichtung 10 zu verkürzen oder zu verlängern und somit die Abdeckung 8 anzuheben oder abzusenken.

Wie sich aus den Figuren 1 und 2 ergibt, ist im Ausführungsbeispiel (optional) vorgesehen, dass die Aufhängeeinrichtung 10 eine Mehrzahl von Ketten 16 aufweist, die unmittelbar oder über ein Verbindungselement an der Abdeckung 8 festgelegt sind, um die Abdeckung 8 in Richtung auf die tragende Konstruktion nach oben zu spannen.

Die Verwendung von Ketten 16 hat sich hierzu als besonders geeignet herausgestellt, insbesondere auch in Kombination mit den nicht näher dargestellten Gewindestangen, mit denen die Ketten 16 angehoben oder weiter abgesenkt werden können.

Im Ausführungsbeispiel nach den Figuren 1 bis 3 ist vorgesehen, dass die Abdeckung 8 mit einem Befestigungsring 17 versehen ist, an dem die Aufhängeeinrichtung 10 angreift. Vorzugsweise weist der Befestigungsring 17 dabei eine Mehrzahl von vorzugsweise gleichmäßig zueinander beabstandet angeordneten Befestigungspunkten 18 auf, an denen die Aufhängeeinrichtung 10, vorzugsweise die Ketten 16, festgelegt werden können. Diese Ausgestaltung hat sich als besonders geeignet herausgestellt, ist jedoch im Rahmen des Ausführungsbeispiels optional. Der Befestigungsring 17 kann mit bekannten Maßnahmen an der durch die Metallbahnen 6 (oder Metallplatten 7) gebildeten Abdeckung 8 gasdicht festgelegt werden, wozu auch entsprechende, nicht näher dargestellte Dichtungen vorgesehen sein können.

In der Figur 4 ist eine zu Figur 3 alternative Konstruktion der Abdeckung 8 dargestellt. Vorgesehen ist dabei, dass die Abdeckung 8 einen äußeren Abdeckungsring 19 mit einer inneren Öffnung 20 aufweist. Die Abdeckung 8 weist ferner eine innere Abdeckscheibe 21 auf, welche die innere Öffnung 20 des äußeren Abdeckungsrings 19 gasdicht verschließt. Im Ausführungsbeispiel verbleibt dabei jedoch eine Rührwerksdurchführung 15, die erst nach dem Einsetzen des Rührwerks 13 gasdicht verschlossen ist.

Im Ausführungsbeispiel nach der Figur 4 ist die innere Öffnung 20 des äußeren Abdeckungsrings 19 strichliniert dargestellt, da diese in der dargestellten Ansicht von der inneren Abdeckscheibe 21 verdeckt wird. Die innere Abdeckscheibe 21 ist mit dem äußeren Abdeckungsring 19 gasdicht verbunden, wozu entsprechende Dichtungen eingesetzt werden können. Grundsätzlich wäre es möglich, die innere Abdeckscheibe 21 mit dem äußeren Abdeckungsring 19 zu verschweißen. Im Ausführungsbeispiel ist jedoch eine Verschraubung vorgesehen. Die in Figur 4 dargestellte Konstruktion hat den Vorteil dass, wenn das Rührwerk 13 aus dem Aufnahmeraum 2 entfernt werden soll, nicht die gesamte Abdeckung 8 demontiert werden muss, sondern es ausreichend ist, die innere Abdeckscheibe 21 von dem äußeren Abdeckungsring 19 zu demontieren. Damit liegt die innere Öffnung 20 des äußeren Abdeckungsrings 19 frei, so dass das Rührwerk 13 entnommen werden kann. Die innere Öffnung 20 kann dabei auch als sogenannter Mannlochdeckel ausgebildet werden, um generell einen Zugang in den Aufnahmeraum 2 zu gewähren.

Die innere Abdeckscheibe 21 kann Bohrungen aufweisen, durch die Schrauben 22 gesteckt werden. Dabei kann vorgesehen sein, dass die Schraubenköpfe der Schrauben 22 mit der inneren Abdeckscheibe 21 unverlierbar verschweißt sind. Mittels der Schraube 22 kann die innere Abdeckscheibe besonders vorteilhaft mit dem äußeren Abdeckungsring 19 verbunden werden.

In Figur 4 sind ferner Befestigungspunkte 18 dargestellt, an denen die Aufhängeeinrichtung 10, insbesondere Ketten 16 der Aufhängeeinrichtung 10 angreifen können.

Grundsätzlich kann die in Figur 4 dargestellte Abdeckung 8 auch mehrere Abdeckungsringe aufweisen. Dies ist in Figur 4 durch die strichliniert und punktierte Linie dargestellt.

In Figur 4 sind ferner Bohrungen 23 am äußeren Randbereich der Abdeckung 8 bzw. am äußeren Randbereich des äußeren Abdeckungsring 19 dargestellt, die dazu dient, die Abdeckung 8 an der Wandung 4 festzulegen. Hierzu können vorzugsweise nachfolgend noch näher dargestellte Befestigungselemente 27, insbesondere Schrauben 27, verwendet werden. Die Befestigungselemente 27 werden nachfolgend als Schrauben 27 bezeichnet, es kann sich jedoch um beliebige Befestigungselemente handeln. Entsprechende Bohrungen 23 weist auch die in Fig. 3 dargestellte Abdeckung 8 auf.

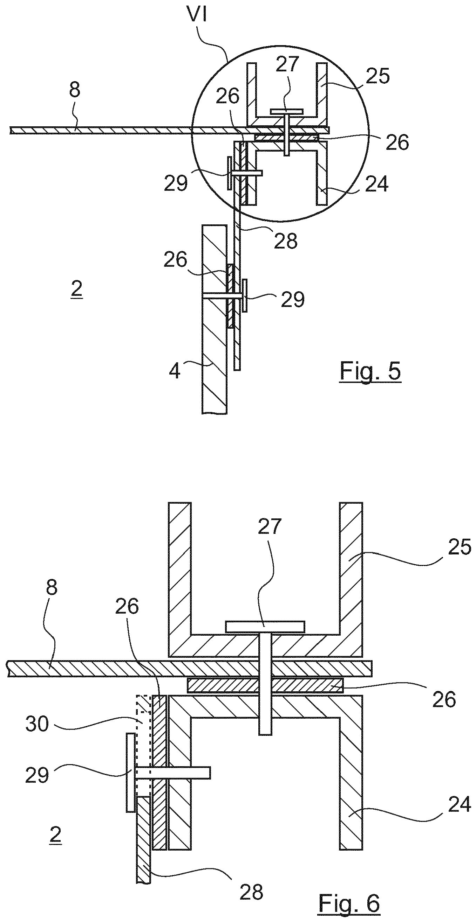

In Figur 5 ist eine besonders geeignete Möglichkeit dargestellt, um die Abdeckung 8 an der Oberseite der Wandung 4 festzulegen. Hierzu ist vorgesehen, dass der umlaufende Randbereich der Abdeckung 8 an seiner Unterseite mit einem unteren Flanschring 24 und an einer Oberseite mit einem oberen Flanschring 25 versehen ist. Die Flanschringe 24, 25 können dabei vorzugsweise miteinander verschraubt werden.

In den Ausführungsbeispielen ist vorzugsweise vorgesehen, dass der umlaufende Randbereich der Abdeckung 8 zwischen dem unteren Flanschring 24 und dem oberen Flanschring 25 kraftschlüssig fixiert, d. h. eingeklemmt wird derart, dass sich eine gasdichte Verbindung ergibt.

Möglich ist auch eine andere kraftschlüssige und/oder eine stoffschlüssige Verbindung, beispielsweise ein Verschweißen. Ein Verschrauben hat sich jedoch als besonders geeignet herausgestellt, insbesondere um zu vermeiden, dass vor Ort auf der Baustelle geschweißt werden muss und insbesondere um die Abdeckung 8 auch wieder lösen zu können. Vorgesehen sein kann eine Dichtung 26 zwischen der Unterseite der Abdeckung 8 und dem unteren Flanschring 24, um an dieser Stelle eine gasdichte Verbindung herzustellen.

Die zur Herstellung der Verbindung, insbesondere der Verschraubung, eingesetzten Befestigungselemente, bei denen es sich vorzugsweise um Schrauben handelt, tragen in Figur 5 das Bezugszeichen 27.

Der im Ausführungsbeispiel dargestellten Behälter 1 ist nicht auf die in Figur 5 dargestellte Ausführungsform der Verbindung zwischen der Abdeckung 8 und der Wandung 4 beschränkt, diese hat sich jedoch als besonders geeignet herausgestellt.

Erfindungsgemäß ist vorgesehen, dass sich die gasdichte Verbindung zwischen der Wandung 4 und der Abdeckung 8 löst, wenn der Druck im Aufnahmeraum 2 einen definierten Berstdruck übersteigt. Der Berstdruck kann dabei vorzugsweise einem Überdruck von 100 mbar, weiter bevorzugt von 80 mbar, besonders bevorzugt von 60 mbar und ganz besonders bevorzugt von 50 mbar, oder weniger als 50 mbar (jedoch vorzugsweise mindestens +10 mbar über dem Betriebsdruck), entsprechen.

Die Verbindung zwischen der Abdeckung 8 und der Wandung 4 wird erfindungsgemäß dadurch gelöst, dass zwischen der Abdeckung 8 und der Wandung 4 des Behälters 1 ein Berstelement 28 eingesetzt ist, welches beim Erreichen des Berstdrucks die gasdichte Verbindung zwischen der Abdeckung 8 und der Wandung 4 löst.

Das Berstelement ist im Ausführungsbeispiel als Berstring 28 ausgebildet, hierauf ist das Ausführungsbeispiel jedoch nicht beschränkt zu verstehen.

Der Berstring 28 kann an einer beliebigen und geeigneten Stelle zwischen der Abdeckung 8 und der Wandung 4 eingesetzt werden. Eine besonders vorteilhafte Konstruktion ist in Figur 5 dargestellt, auf die das Ausführungsbeispiel nicht beschränkt ist.

In dem in Figur 5 dargestellten Ausführungsbeispiel ist vorgesehen, dass der untere Flanschring 24 mit dem Berstring 28 verbunden ist. Des Weiteren ist vorgesehen, dass der Berstring 28 mit der Wandung 4 des gasdichten Behälters 1 verbunden ist.

Um den Berstring 28 mit dem unteren Flanschring 24 bzw. mit der Wandung 4 zu verbinden, sind in dem in Figur 5 dargestellten Ausführungsbeispiel Befestigungselemente 29, z.B. Verschraubungen vorgesehen. Es kann sich bei den Befestigungselemente 29, insbesondere um Schrauben 29 handeln.

Alternativ kann auch eine andere kraftschlüssige oder auch eine stoffschlüssige Verbindung, zum Beispiel eine Verschweißung, vorgesehen sein. Eine Verschraubung hat sich jedoch als besonders geeignet herausgestellt, insbesondere um die Verbindung auch wieder zu lösen.

Zur Verbindung des Berstrings 28 mit der Wandung 4 können ebenfalls Befestigungselemente 29 vorgesehen sein. Vorzugsweise sind die Befestigungselemente 29 durch die Wandung 4 durchgeschraubt, was sich insbesondere für Wandungen 4 aus Metall, insbesondere Stahl, eignet. Alternativ kann auch vorgesehen sein, dass die Befestigungselemente 29 nur in die Wandung 4 eingeschraubt werden, was sich insbesondere für Wandungen 4 aus Beton oder Metallbeton, insbesondere Stahlbeton, eignet.

Der Berstring 28 kann derart ausgebildet sein, dass sich die Verbindung zwischen dem Berstring 28 und der Wandung 4 und/oder zwischen dem Berstring 28 und dem unteren Flanschring 24 löst, wenn der Berstdruck überschritten wird. Dies kann vorzugweise z. B. dadurch erfolgen, dass der Berstring angehoben wird. Eine mögliche vorteilhafte Ausgestaltung hierfür ist in Fig.6 dargestellt. Ferner kann auch vorgesehen sein, dass der Berstring 28 zerstört wird, wenn der Berstdruck überschritten wird.

In dem in Figur 5 dargestellten Ausführungsbeispiel ist, wie beschrieben, auch ein oberer Flanschring 25 dargestellt, der sich grundsätzlich eignet, um eine stabile Verbindung herzustellen, dies ist jedoch im Rahmen des Ausführungsbeispiels nach Figur 5 grundsätzlich optional. In Figur 5 ist vorgesehen, dass an den Verbindungsstellen Dichtungen 26 eingesetzt werden, damit der Aufnahmeraum 2 gasdicht verschlossen ist.

Die Flanschringe 24, 25 sind vorzugsweise im Querschnitt betrachtet als U-Profile ausgebildet.

Das in Figur 5 exemplarisch dargestellte Ausführungsbeispiel eignet sich für Wandungen 4 aus beliebigen Materialien, vorzugsweise für Wandungen 4 aus Metall, insbesondere Stahl, Beton, Metallbeton, insbesondere Stahlbeton. Insbesondere ist es möglich, die Abdeckung 8 nachträglich (vor Ort) auf die bereits bestehende Wandung 4 aufzubringen. Mit der erfindungsgemäßen Lösung können ferner auch bereits bestehende Behälter nachträglich mit einem Berstelement bzw. einem Berstring 28 nachgerüstet werden.

Figur 6 zeigt eine mögliche Ausgestaltung, um ein Anheben der Abdeckung 8 von der Wandung 4 zu ermöglichen. In Figur 6 ist hierzu dargestellt, dass der Berstring 28 zur Verbindung mit dem unteren Flanschring 24 Langlöcher 30 aufweist, durch welches die Befestigungselemente 29 geführt sind, um den Berstring 28 mit dem unteren Flanschring 24 zu verbinden. Vorgesehen ist dabei, dass im normalen Betriebszustand, d. h. wenn in dem gasdichten Behälter 1 der Betriebsdruck herrscht, durch die Befestigungselemente 29 eine gasdichte Verbindung zwischen dem unteren Flanschring 24 und dem Berstring 28 hergestellt ist, vorzugsweise unter Verwendung einer Dichtung 26.

Die Verbindung zwischen dem Bestring 28 und dem unteren Flanschring 24 ist derart beschaffen, dass sich die Befestigungselemente 29 in dem Langloch 30 bewegen, im Ausführungsbeispiel nach oben, wenn der Berstdruck erreicht wird.

Die Gestaltung des Berstdruck führt dazu, dass sich aufgrund des gestiegenen Drucks im Behälter 1 die Befestigungselemente 29 in den Langlöchern 30 nach oben bewegen, wodurch sich auch der untere Flanschring 24 entsprechend nach oben bewegt. Somit kann sich auch Abdeckung 8 von der Wandung 4 nach oben bewegen.

Da der Berstring 28 mit dem unteren Flanschring 24 über die Langlöcher 30 und die durch die Langlöcher 30 durchgeführten Befestigungselemente 29 verbunden ist, löst sich gasdichte Verbindung zwischen dem Berstring 28 und dem unteren Flanschring 24 wenn der Berstdruck erreicht wird.

Es ist im Ausführungsbeispiel vorgesehen, dass sich durch die Relativbewegung zwischen den Befestigungselementen 29 und den Langlöchern 30 eine Position ergibt, in der Gas aus dem Behälter 1 ausströmen kann. Es kann auch vorgesehen sein, dass die Bewegung dazu führt, dass sich die Abdeckung 8 vollständig von der Wandung 4 (aufgrund der gelösten Verbindung zwischen dem unteren Flanschring 24 und dem Berstring 28) löst, so dass Gas aus dem Behälter 1 ausströmen kann.

Es kann vorgesehen sein, dass das Langloch 30 derart beschaffen ist, dass sich der untere Flanschring 24 von dem Berstring 28 löst, wenn die Befestigungselemente 29 eine obere Position innerhalb der Langlöcher 30 erreicht haben.

Ergänzend oder alternativ zu einer Ausbildung von Langlöchern 30 können auch Schlitze in dem Berstring 28 vorhanden sein, die eine analoge Bewegung ermöglichen. Die Schlitze stellen dabei im Wesentlichen nach oben geöffnete Langlöcher 30 dar. Dies ist in Figur 6 dadurch symbolisiert, dass das obere Ende des Berstrings 28 strichliniert dargestellt ist, d. h. die Langlöcher 30 können als Schlitze ausgeführt bzw. nach oben offen ausgebildet sein. Dadurch kann sich die Abdeckung 8 ohne Begrenzung durch die Langlöcher 30 nach oben anheben. Beim Erreichen des Berstdrucks kann sich die Abdeckung 8 somit, vorzugsweise vollständig, von der Wandung 4 abheben.

Eine entsprechende Verbindung über Langlöcher 30 und/oder Schlitze kann auch zwischen dem Berstring 28 und der Wandung 4 vorgesehen sein, d. h. an der Position, an der in Figur 5 ebenfalls Befestigungselemente 29 eingezeichnet sind.

Es kann auch vorgesehen sein, dass Langlöcher 30 und/oder Schlitze sowohl zur Verbindung des Berstrings 28 mit der Wandung 4 als auch zur Verbindung des Berstrings 28 mit dem unteren Flanschring 24 vorgesehen sind.

Ferner ist es im Rahmen der Erfindung auch möglich, dass der untere Flanschring 24 über nicht näher dargestellte Langlöcher verfügt. In diesem Fall kann auf die Ausbildung von Langlöchern in dem Berstring 28 ggf. verzichtet werden. Auch eine derartige Ausgestaltung führt dazu, dass sich die Abdeckung 8 von der Wandung 4 abheben kann bzw. dass sich eine Relativbewegung zwischen den Befestigungselementen 29 und den Langlöchern 30 derart ergibt, dass die Abdeckung 8 von der Wandung 4 abgehoben wird, d. h. der untere Flanschring 24 nach oben bewegt wird.

Eine Verbindung zwischen dem Berstring 28 und der Wandung 4 bzw. dem unteren Flanschring 24 derart, dass diese im Betriebszustand gasdicht ist, sich jedoch beim Erreichen des Berstdrucks die gasdichte Verbindung öffnet, lässt sich in einfacher Weise erreichen, beispielsweise dadurch, dass entsprechende Anziehdrehmomente bei den Befestigungselementen 29, bei denen es sich vorzugsweise um Schrauben handelt, vorgesehen sind, oder geeignete Maßnahmen ergriffen werden, durch die gewährleistet ist, dass der Kraft- und/oder Reibschluss zwischen dem Berstring 28 und der Wandung 4 bzw. dem unteren Flanschring 24 geringer ist als der Druck, der zu einem Anheben der Abdeckung 8 führt, wenn der Berstdruck in dem gasdichten Behälter 1 erreicht ist. Im Ausführungsbeispiel ist vorgesehen, dass der Berstdruck einem Überdruck von wenigstens 30 mbar, vorzugsweise wenigstens 40 mbar, weiter bevorzugt wenigstens 50 mbar, ganz besonders bevorzugt wenigstens 60 mbar, insbesondere wenigstens 80 mbar, entspricht.

Weiter ist im Ausführungsbeispiel vorzugsweise vorgesehen, dass der Berstdruck einem Überdruck von höchstens 200 mbar, vorzugsweise höchstens 150 mbar, weiter bevorzugt höchstens 120 mbar, noch weiter bevorzugt höchstens 100 mbar, ganz besonders bevorzugt 80 mbar, insbesondere höchstens 50 mbar, entspricht.

Die Höchstwerte haben sich insbesondere in Kombination mit den vorgenannten Mindestwerten als besonders geeignet herausgestellt, wobei in diesem Fall die Werte derart auszuwählen sind, dass der Mindestwert geringer ist als der Höchstwert. Ganz besonders vorteilhaft ist dabei ein Höchstwert von 50 mbar.

Der nachfolgend dargestellte Wertebereich hat sich für den Berstdruck im Ausführungsbeispiel als besonders geeignet herausgestellt. Es eignet sich in besonderer Weise, wenn der Berstdruck einem Überdruck zwischen 30 und 150 mbar, vorzugsweise zwischen 30 und 100 mbar, weiter bevorzugt zwischen 30 und 80 mbar, noch weiter bevorzugt zwischen 30 und 60 mbar, ganz besonders bevorzugt zwischen 30 und 50 mbar insbesondere zwischen 40 und 50 mbar, entspricht.

Im Ausführungsbeispiel ist vorgesehen, dass sich durch den Berstring 28 die Verbindung zwischen der Wandung 4 und der Abdeckung 8 vollständig löst, so dass großvolumig Gas austreten kann, nicht nur durch eine kleine Öffnung.

Im Ausführungsbeispiel ist vorgesehen, dass der Betriebsdruck des gasdichten Behälters 1 zwischen 20 und 29 mbar, vorzugsweise bei 25 mbar, liegt.

Der Berstring 28 ist vorzugsweise derart gestaltet, dass die ganze Abdeckung 8 wie ein Deckel von der Wandung 4 abgehoben wird, wenn der Berstdruck einen definierten Wert übersteigt.

## Patentansprüche

1. Gasdichter Behälter (1), insbesondere Silo zur Lagerung von organischen Substanzen, aufweisend einen Aufnahmeraum (2), einen Boden (3), eine umlaufende Wandung (4) und eine Dacheinrichtung (5), wobei die Dacheinrichtung (5) eine gasdichte Abdeckung (8) aufweist, welche mit der Wandung (4) gasdicht verbunden ist,
**dadurch gekennzeichnet, dass**
zwischen der Abdeckung (8) und der Wandung (4) des Behälters (1) ein Berstelement (28) eingesetzt ist, welches beim Erreichen eines definierten Berstdrucks die gasdichte Verbindung zwischen der Abdeckung (8) und der Wandung (4) löst.

2. Gasdichter Behälter (1) nach Anspruch 1,
**dadurch gekennzeichnet, dass**
der Berstdruck einem Überdruck von wenigstens 30 mbar, vorzugsweise wenigstens 40 mbar, weiter bevorzugt wenigstens 50 mbar, ganz besonders bevorzugt wenigstens 60 mbar, insbesondere wenigstens 80 mbar, entspricht.

3. Gasdichter Behälter (1) nach Anspruch 1 oder 2,
**dadurch gekennzeichnet, dass**
der Berstdruck einem Überdruck von höchstens 200 mbar, vorzugsweise höchstens 150 mbar, weiter bevorzugt höchstens 120 mbar, noch weiter bevorzugt höchstens 100 mbar, ganz besonders bevorzugt 80 mbar, insbesondere höchstens 50 mbar, entspricht.

4. Gasdichter Behälter (1) nach einem der Ansprüche 1, 2 oder 3,
**dadurch gekennzeichnet, dass**
der Berstdruck einem Überdruck zwischen 30 und 150 mbar, vorzugsweise zwischen 30 und 100 mbar, weiter bevorzugt zwischen 30 und 80 mbar, noch weiter bevorzugt zwischen 30 und 60 mbar, ganz besonders bevorzugt zwischen 30 und 50 mbar insbesondere zwischen 40 und 50 mbar, entspricht.

5. Gasdichter Behälter (1) nach einem der Ansprüche 1 bis 4,
**dadurch gekennzeichnet, dass**
das Berstelement (28) als Berstring ausgebildet ist.

6. Gasdichter Behälter (1) nach einem der Ansprüche 1 bis 5,
**dadurch gekennzeichnet, dass**
ein umlaufender Randbereich der Abdeckung (8) an einer Unterseite mit einem unteren Flanschring (24) und/oder an einer Oberseite mit einem oberen Flanschring (25) versehen ist.

7. Gasdichter Behälter (1) nach Anspruch 6,
**dadurch gekennzeichnet, dass**
der untere Flanschring (24) mit dem Berstring (28) und der Berstring (28) mit der Wandung (4) des Behälters (1) verbunden ist.

8. Gasdichter Behälter (1) nach einem der Ansprüche 1 bis 7,
**dadurch gekennzeichnet, dass**
die Verbindung zwischen dem Berstring (28) und der Wandung (4) und/oder dem unteren Flanschring (24) Langlöcher (30) und/oder Schlitze aufweist, durch welche Befestigungselemente (29) geführt sind, um den Berstring (28) mit der Wandung (4) und/oder dem unteren Flanschring (24) zu verbinden.

9. Gasdichter Behälter (1) nach Anspruch 8,
**dadurch gekennzeichnet, dass**
die Verbindung zwischen dem Berstring (28) und der Wandung (4) und/oder dem unteren Flanschring (24) derart beschaffen ist, dass die Verbindung in einem Betriebszustand des gasdichten Behälters (1) gasdicht ist, jedoch beim Erreichen des Berstdrucks eine Relativbewegung zwischen dem Berstring (28) und den Langlöchern (30) und/oder den Schlitzen stattfindet, die dazu führt, dass sich die Abdeckung (8) von der Wandung (4) abhebt.

10. Gasdichter Behälter (1) nach einem der Ansprüche 1 bis 9,
**dadurch gekennzeichnet, dass**
die Dacheinrichtung (5) eine oberhalb der Abdeckung (8) angeordnete tragende Konstruktion (9) und eine Aufhängeeinrichtung (10) aufweist, wobei die Aufhängeeinrichtung (10) an der Abdeckung (8) und an der tragenden Konstruktion (9) festgelegt ist und die Aufhängeeinrichtung (10) ausgebildet ist, um die Abdeckung (8) in Richtung auf die tragende Konstruktion (9) nach oben zu verspannen.

11. Gasdichter Behälter (1) nach Anspruch 10,
**dadurch gekennzeichnet, dass**
die Aufhängeeinrichtung (10) mittig oberhalb der Abdeckung (8), vorzugsweise koaxial zu der vertikal verlaufenden Mittelachse des Behälters (1) angeordnet ist.

12. Gasdichter Behälter (1) nach einem der Ansprüche 10 oder 11,
**dadurch gekennzeichnet, dass**
die Aufhängeeinrichtung (10) eine Mehrzahl von Ketten (16) aufweist, die unmittelbar oder über ein Befestigungselement, vorzugsweise einen Befestigungsring (17), an der Abdeckung (8) festgelegt sind, um die Abdeckung (8) in Richtung auf die tragende Konstruktion (9) nach oben zu spannen.

13. Gasdichter Behälter (1) nach einem der Ansprüche 10 bis 12,
**dadurch gekennzeichnet, dass**
die Aufhängeeinrichtung (10) ausgebildet ist, um die Abdeckung (8) innerhalb eines definierten Hubs anzuheben und abzusenken.

14. Gasdichter Behälter (1) nach einem der Ansprüche 1 bis 13,
**dadurch gekennzeichnet, dass**
der Aufnahmeraum (2) ein Volumen von 100 bis 50.000 m³, vorzugsweise von 1.000 bis 10.000 m³, besonders bevorzugt von 2.000 bis 8.000 m³ aufweist.

15. Vorrichtung zur Gewinnung eines gasförmigen Energieträgers aus organischen Substanzen, insbesondere Biogasreaktor, aufweisend einen gasdichten Behälter (1) nach einem der Ansprüche 1 bis 14.

## Claims

1. Gas-tight container (1), in particular a silo for storing organic substances, comprising a receiving space (2), a base (3), a peripheral wall (4) and a roof means (5), the roof means (5) having a gas-tight cover (8) which is connected in a gas-tight manner to the wall (4),
**characterized in that**
a bursting element (28) is inserted between the cover (8) and the wall (4) of the container (1), which bursting element (28) releases the gas-tight connection between the cover (8) and the wall (4) when a defined bursting pressure is reached.

2. Gas-tight container (1) according to claim 1,
**characterized in that**
the burst pressure corresponds to an overpressure of at least 30 mbar, preferably at least 40 mbar, more preferably at least 50 mbar, very particularly preferably at least 60 mbar, in particular at least 80 mbar.

3. Gas-tight container (1) according to claim 1 or 2,
**characterized in that**
the burst pressure corresponds to an overpressure of at most 200 mbar, preferably at most 150 mbar, further preferably at most 120 mbar, still further preferably at most 100 mbar, very particularly preferably 80 mbar, in particular at most 50 mbar.

4. Gas-tight container (1) according to one of claims 1, 2 or 3,
**characterized in that**
the bursting pressure corresponds to an overpressure between 30 and 150 mbar, preferably between 30 and 100 mbar, more preferably between 30 and 80 mbar, still more preferably between 30 and 60 mbar, very particularly preferably between 30 and 50 mbar, in particular between 40 and 50 mbar.

5. Gas-tight container (1) according to one of claims 1 to 4,
**characterized in that**
the bursting element (28) is designed as a bursting ring.

6. Gas-tight container (1) according to one of claims 1 to 5,
**characterized in that**
a circumferential edge region of the cover (8) is provided on a lower side with an lower flange ring (24) and/or on an upper side with an upper flange ring (25).

7. Gas-tight container (1) according to claim 6,
**characterized in that**
the lower flange ring (24) is connected to the bursting ring (28) and the bursting ring (28) is connected to the wall (4) of the container (1).

8. Gas-tight container (1) according to one of the claims 1 to 7,
**characterized in that**
the connection between the bursting ring (28) and the wall (4) and/or the lower flange ring (24) has elongated holes (30) and/or slots through which fastening elements (29) are guided in order to connect the bursting ring (28) to the wall (4) and/or the lower flange ring (24).

9. Gas-tight container (1) according to claim 8,
**characterized in that**
the connection between the bursting ring (28) and the wall (4) and/or the lower flange ring (24) is such that the connection is gas-tight in an operating state of the gas-tight container (1), but when the bursting pressure is reached, a relative movement takes place between the bursting ring (28) and the elongated holes (30) and/or the slots, which results in the cover (8) lifting off from the wall (4).

10. Gas-tight container (1) according to one of the claims 1 to 9,
**characterized in that**
the roof means (5) has a supporting structure (9) arranged above the cover (8) and a suspension means (10), the suspension means (10) being fixed to the cover (8) and to the supporting structure (9) and the suspension means (10) being designed to brace the cover (8) upwards in the direction of the supporting structure (9).

11. Gas-tight container (1) according to claim 10,
**characterized in that**
the suspension means (10) is arranged centrally above the cover (8), preferably coaxially to the vertically extending central axis of the container (1).

12. Gas-tight container (1) according to one of claims 10 or 11,
**characterized in that**
the suspension means (10) comprises a plurality of chains (16) which are fixed to the cover (8) directly or via a fastening element, preferably a fastening ring (17), in order to brace the cover (8) upwards in the direction of the supporting structure (9).

13. Gas-tight container (1) according to any one of claims 10 to 12,
**characterized in that**
the suspension means (10) is designed to raise and lower the cover (8) within a defined stroke.

14. Gas-tight container (1) according to one of claims 1 to 13,
**characterized in that**
the receiving space (2) has a volume of from 100 to 50,000 m³, preferably from 1,000 to 10,000 m³, more preferably from 2,000 to 8,000 m³.

15. Apparatus for obtaining a gaseous energy carrier from organic substances, in particular biogas reactor, comprising a gas-tight container (1) according to one of claims 1 to 14.

## Revendications

1. Conteneur étanche aux gaz (1), notamment silo pour le stockage de substances organiques, ayant un espace de réception (2), un fond (3), une paroi périphérique (4) et un dispositif de toit (5), dans lequel le dispositif de toit (5) a un couvercle étanche aux gaz (8) qui est relié de manière étanche aux gaz avec la paroi (4),
**caractérisé en ce que**,
un élément de rupture (28) est inséré entre le couvercle (8) et la paroi (4) du conteneur (1) pour supprimer la liaison étanche aux gaz entre le couvercle (8) et la paroi (4) lorsqu'une pression de rupture définie est atteinte.

2. Conteneur étanche aux gaz (1) selon la revendication 1,
**caractérisé en ce que**,
la pression de rupture correspond à une surpression d'au moins 30 mbar, de préférence d'au moins 40 mbar, de manière plus préférentielle d'au moins 50 mbar, de manière particulièrement préférentielle d'au moins 60 mbar, notamment d'au moins 80 mbar.

3. Conteneur étanche aux gaz (1) selon la revendication 1 ou 2,
**caractérisé en ce que**,
la pression de rupture correspond à une surpression de 200 mbar au maximum, de préférence de 150 mbar au maximum, de manière plus préférentielle de 120 mbar au maximum, de manière encore plus préférentielle de 100 mbar au maximum, de manière particulièrement préférentielle de 80 mbar au maximum, notamment de 50 mbar au maximum.

4. Conteneur étanche aux gaz (1) selon l'une des revendications 1, 2 ou 3,
**caractérisé en ce que**,
la pression de rupture correspond à une surpression entre 30 et 150 mbar, de préférence entre 30 et 100 mbar, de manière plus préférentielle entre 30 et 80 mbar, de manière encore plus préférentielle entre 30 et 60 mbar, de manière particulièrement préférentielle entre 30 et 50 mbar, notamment entre 40 et 50 mbar.

5. Conteneur étanche aux gaz (1) selon l'une des revendications 1 à 4,
**caractérisé en ce que**,
l'élément de rupture (28) est réalisé sous la forme d'un anneau de rupture.

6. Conteneur étanche aux gaz (1) selon l'une des revendications 1 à 5,
**caractérisé en ce que**,
une zone de bord périphérique du couvercle (8) est pourvue sur une face inférieure d'un anneau-bride inférieur (24) et/ou sur une face supérieure d'un anneau-bride supérieur (25).

7. Conteneur étanche aux gaz (1) selon la revendication 6,
**caractérisé en ce que**,
l'anneau-bride inférieur (24) est relié à l'anneau de rupture (28) et l'anneau de rupture (28) est relié à la paroi (4) du conteneur (1).

8. Conteneur étanche aux gaz (1) selon l'une des revendications 1 à 7,
**caractérisé en ce que**,
la liaison entre l'anneau de rupture (28) et la paroi (4) et/ou l'anneau-bride inférieur (24) comporte des trous oblongs (30) et/ou des fentes, à travers lesquels passent des éléments de fixation (29) pour relier l'anneau de rupture (28) à la paroi (4) et/ou à l'anneau-bride inférieur (24).

9. Conteneur étanche aux gaz (1) selon la revendication 8,
**caractérisé en ce que**,
la liaison entre l'anneau de rupture (28) et la paroi (4) et/ou l'anneau-bride inférieur (24) est ménagée de sorte que la liaison, dans un état de fonctionnement du conteneur étanche aux gaz (1), est étanche aux gaz, et que, cependant, lorsque la pression de rupture est atteinte, un mouvement relatif s'effectue entre l'anneau de rupture (28) et les trous oblongs (30) et/ou les fentes, provoquant le soulèvement du couvercle (8) par rapport à la paroi (4).

10. Conteneur étanche aux gaz (1) selon l'une des revendications 1 à 9,
**caractérisé en ce que**,
le dispositif de toit (5) comporte une structure porteuse (9) disposée au-dessus du couvercle (8) et un dispositif de suspension (10), dans lequel le dispositif de suspension (10) est fixé au couvercle (8) et à la structure porteuse (9) et le dispositif de suspension (10) est formé de sorte à tendre le couvercle (8) vers le haut en direction de la structure porteuse (9).

11. Conteneur étanche aux gaz (1) selon la revendication 10,
**caractérisé en ce que**,
le dispositif de suspension (10) est disposé de manière centrée au-dessus du couvercle (8), de préférence de manière coaxiale à l'axe central vertical du conteneur (1).

12. Conteneur étanche aux gaz (1) selon l'une des revendications 10 ou 11,
**caractérisé en ce que**,
le dispositif de suspension (10) comporte une pluralité de chaînes (16) fixées au couvercle (8) directement ou par l'intermédiaire d'un élément de fixation, de préférence un anneau de fixation (17), pour tendre le couvercle (8) vers le haut en direction de la structure porteuse (9).

13. Conteneur étanche aux gaz (1) selon l'une des revendications 10 à 12,
**caractérisé en ce que**,
le dispositif de suspension (10) est conçu pour soulever et abaisser le couvercle (8) à l'intérieur d'une course définie.

14. Conteneur étanche aux gaz (1) selon l'une des revendications 1 à 13,
**caractérisé en ce que**,
l'espace de réception (2) a un volume de 100 à 50.000 m³, de préférence de 1.000 à 10.000 m³, de manière particulièrement préférentielle de 2.000 à 8.000 m³.

15. Dispositif de récupération d'une ressource énergétique gazeuse à partir de substances organiques, notamment réacteur à biogaz, équipé d'un conteneur étanche aux gaz (1) selon l'une des revendications 1 à 14.
